# FASCICULE DE BREVET EUROPEEN

(11) **EP 0 659 216 B1**
(45) Date de publication et mention de la délivrance du brevet: **08.03.2006**
(21) Numéro de dépôt: 94921008.2
(22) Date de dépôt: 30.06.1994
(51) Int. Cl.: C12N 15/86, C12N 5/10, C12N 5/16, C12N 5/22

(54) **VECTEUR RETROVIRAL POUR LE TRANSFERT ET L'EXPRESSION DE GENES A VISEE THERAPEUTIQUE, DANS DES CELLULES EUCARYOTES**
RETROVIRALER VEKTOR ZUM TRANSFER UND ZUR EXPRESSION VON THERAPEUTISCHEN GENEN IN EUKARYOTEZELLEN
RETROVIRAL VECTOR FOR THE TRANSFER AND EXPRESSION OF GENES FOR THERAPEUTICAL PURPOSES IN EUCARYOTIC CELLS

(30) Priorité: 30.06.1993 FR 9308015
(43) Date de publication de la demande: 28.06.1995
(73) Titulaire: Cohen-Haguenauer, Odile, F-75116 Paris (FR)
(72) Inventeur: Cohen-Haguenauer, Odile, F-75116 Paris (FR)
(74) Mandataire: Breese, Pierre
(86) Numéro de dépôt international: PCT/FR1994/000806
(87) Numéro de publication internationale: WO 1995/001447

(56) Documents cités:
- WO-A-89/11539
- WO-A-90/06757
- Genbank database ,Bethesda, US GenBank ACC. NO. (GBN): Z22761 Bestwick,R. et al. Retroviral expression vector... & PROCEEDINGS OF THE NATIONAL ACADEMY OF SCIENCES OF USA., vol.85, no.15, 1988, WASHINGTON US pages 5404 - 5408 BESTWICK R.K. ET AL.
- Genbank Databank Bethesda GenBank ACC. NO. (GBN): M60088 Yu,X.-F et al. Synthetic plasmid p806 polylinker site.

## Description

L'invention a pour objet de nouveaux vecteurs rétroviraux, notamment pour le transfert et l'expression de gènes dans des cellules eucaryotes. L'invention propose à cet égard des vecteurs particulièrement appropriés pour l'application au transfert de gènes à des fins cliniques thérapeutiques, prophylactiques ou diagnostiques.

L'évolution rapide de la génétique moléculaire a permis l'identification d'un nombre croissant d'anomalies moléculaires responsables de maladies humaines. Au sein de l'unité fonctionnelle que constitue un gène, se juxtaposent des régions responsables de l'expression d'un signal biologique et de sa régulation. Chacune de ces régions est susceptible d'être le siège de modifications pathologiques conduisant à une anomalie de synthèse quantitative ou qualitative. La détection de ces anomalies en autorise un dépistage mais l'objectif prioritaire reste d'ordre thérapeutique.

Le transfert de gènes à visée thérapeutique ou "thérapie génique" somatique consiste à insérer un gène "réparateur" dans les cellules somatiques d'un organisme constitué, pour pallier au dysfonctionnement d'un gène endogène ; voire y ajouter une fonction nouvelle dans un but thérapeutique. Le changement génétique résultant est susceptible de se transmettre aux cellules filles de la cellule manipulée, mais il ne sera pas héréditaire. La contrepartie normale de séquences d'ADN altérées se voit ainsi transformée en médicament.

Le domaine de la thérapie génique est aujourd'hui en pleine évolution et conjugue des essais cliniques (pour des effectifs de patients encore très limités) avec des travaux de recherche des plus fondamentaux, tels que les modes de régulation de l'expression génique ou la vectorisation des séquences thérapeutiques d'acides nucléiques. Les vecteurs actuellement utilisés sont dérivés soit de virus inactivés, tels que les rétrovirus ou les adénovirus, soit de complexes macromoléculaires. Les rétrovirus s'adressent plus volontiers à un tissu cible comprenant un contingent de cellules souches susceptibles d'être manipulées ex vivo ; par contre, lorsque le tissu cible est constitué de cellules en différenciation terminale, ou bien intriqué dans un organe dont les contraintes architecturales ont des conséquences fonctionnelles majeures, comme le poumon, le transfert de gènes doit s'opérer in vivo, par exemple au moyen d'adénovirus. La thérapie génique trouve ses applications dans des pathologies aussi diverses que les maladies héréditaires dues à l'altération d'un seul gène, comme la myopathie de Duchenne, les maladies lysosomiales, la mucoviscidose ou des maladies acquises telles que le sida, les cancers, la maladie thrombo-embolique ou des maladies neurologiques dégénératives, les maladies hématologiques constitutionnelles.

Néanmoins, si les applications potentielles du transfert de gènes sont extraordinairement larges, les développements thérapeutiques de cette approche et sa pertinence se heurtent encore à des difficultés technologiques.

Le développement de vecteurs rétroviraux plus efficaces que les outils existant constitue à cet égard un objectif prioritaire. En effet, les vecteurs rétroviraux ont fait la preuve de leur efficacité dans des systèmes où les cellules cibles du transfert sont classiquement l'objet de mitoses et comportent idéalement un contingent de cellules souches ; mais les limitations sont essentiellement liées à une infectivité insuffisante des virus utilisés et/ou un niveau de transcription trop modeste. Dans ce but, des vecteurs intéressants peuvent être sélectionnés en considérant notamment leur titre infectieux.

L'invention a pour but de proposer des vecteurs plus performants que ceux existant, lesquels sont actuellement pour la plupart dérivés du squelette du virus de la leucémie murine de Moloney.

L'invention repose sur des travaux réalisés à partir d'une souche particulièrement virulente du virus de Friend. L'isolat I-5 du virus écotrope de la leucémie murine de Friend a été obtenu à partir de cultures de moelle à long terme infectées par le complexe viral inducteur de polyglobulies du virus de Friend (FV-P) (Mathieu-Mahul et al., 1982). La souche FB29 du F-MuLV dérivée de l'isolat I-5 (Sitbon et al, 1986) est responsable d'effets cytolytiques et leucémogènes sur les cellules érythroïdes, conduisant à une anémie hémolytique précoce et sévère suivie d'une érythroleucémie tardive chez les souris susceptibles, inoculées à la naissance. Les régions responsables de l'érythroleucémie ont été localisées au niveau de la région U3 du LTR viral (Sitbon et al, 1986 ; Sitbon et al, 1991). Le déterminant principal de l'anémie hémolytique semble dépendre de séquences d'enveloppe spécifiques de la souche FB29 ; la sévérité pouvant en être influencée par trois régions distinctes, dont un segment structural de l'enveloppe, des séquences activatrices de la transcription localisées dans la région U3, et enfin, des séquences des régions U5-gag-pol (Sitbon et al., 1990).

En outre, des analyses en microscopie électronique des particules virales ont authentifié une capacité d'encapsidation significativement supérieure (1,5 à 2 log).

Les inventeurs se sont intéressés aux caractéristiques particulières de cette souche FB29 et l'ont utilisée pour définir des vecteurs rétroviraux.

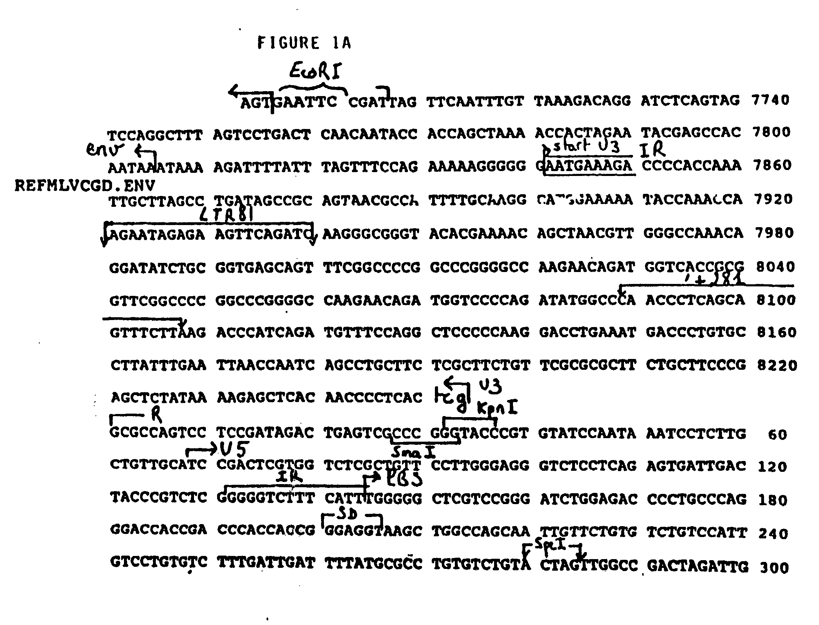

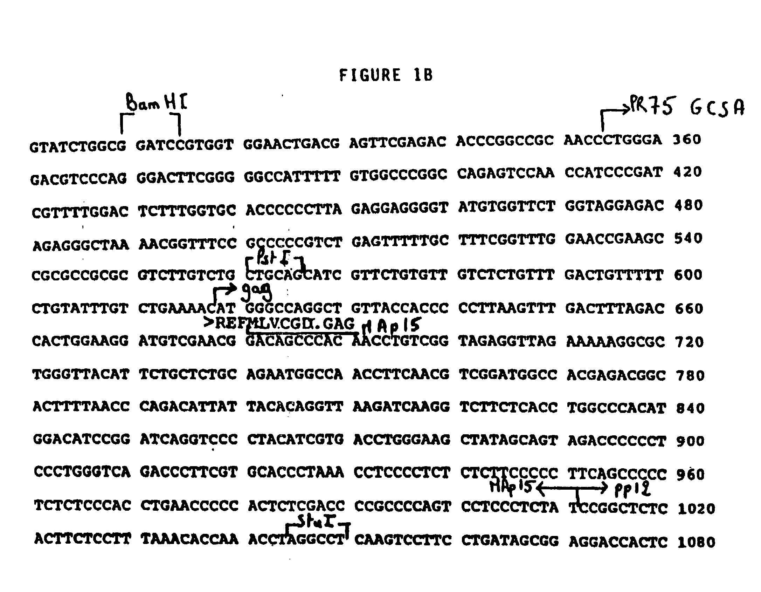

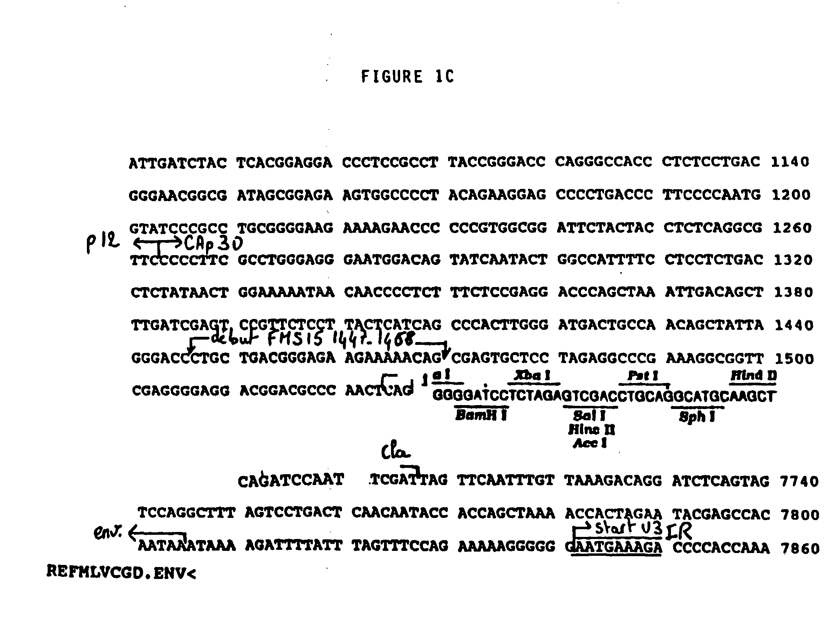

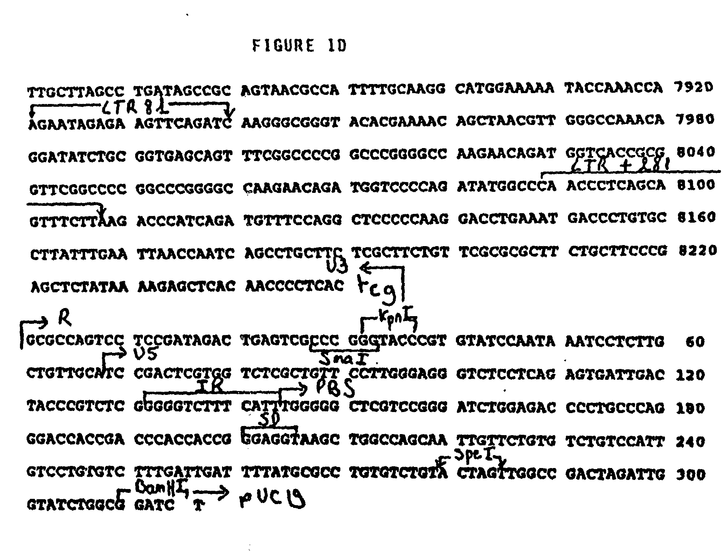

Selon un premier mode de réalisation, l'invention a pour objet un vecteur rétroviral recombinant pour le clonage et/ou l'expression et/ou le transfert d'une séquence de nucléotides exogène, caractérisé en ce qu'il comprend toute séquence contenue dans le fragment ClaI-PvuII situé environ entre les nucléotides 7702 et 1527 de la séquence donnée à la figure 1 et comprenant l'enchaînement LTR compris entre les nucléotides 7842 et 144, le site PBS débutant au nucléotide 145, la séquence d'encapsidation comprise dans l'enchaînement de 250 nucléotides suivant la fin de la séquence LTR, ladite séquence étant capable de contrôler le clonage et/ou l'expression et/ou le transfert de la séquence exogène.

Selon un autre mode de réalisation de l'invention, le vecteur recombinant est caractérisé en ce qu'il comprend toute séquence contenue dans le fragment ClaI-BamHI comprenant les nucléotides 7702 à 310 de la séquence représentée à la figure 1, et ladite séquence comprenant l'enchaînement LTR compris entre les nucléotides 7842 et 144, le site PBS débutant au nucléotide 145, la séquence d'encapsidation comprise dans l'enchaînement de 250 nucléotides suivant la fin de la séquence LTR, ladite séquence étant capable de contrôler l'expression et/ou le transfert de la séquence exogène, quelle que soit son orientation transcriptionnelle par rapport à l'orientation transcriptionnelle du virus.

Selon ce deuxième mode de réalisation de l'invention, le vecteur est donc un vecteur rétroviral pour l'expression et/ou le transfert d'une séquence de nucléotides exogène comprenant toute séquence contenue dans le fragment ClaI-BamHI situé environ entre les nucléotides 7702 et 310 de la séquence donnée à la figure 1, ladite séquence ayant la capacité de contrôler le clonage et/ou l'expression et/ou le transfert de la séquence exogène.

Les sites ClaI et BamHI dont il est question ci-dessus ont leur origine dans la souche FB29.

Lors de la construction des vecteurs rétroviraux ou des vecteurs destinés à l'obtention de lignées d'encapsidation, ces sites peuvent être modifiés et notamment remplacés, créant éventuellement des sites de coupure enzymatique distincts.

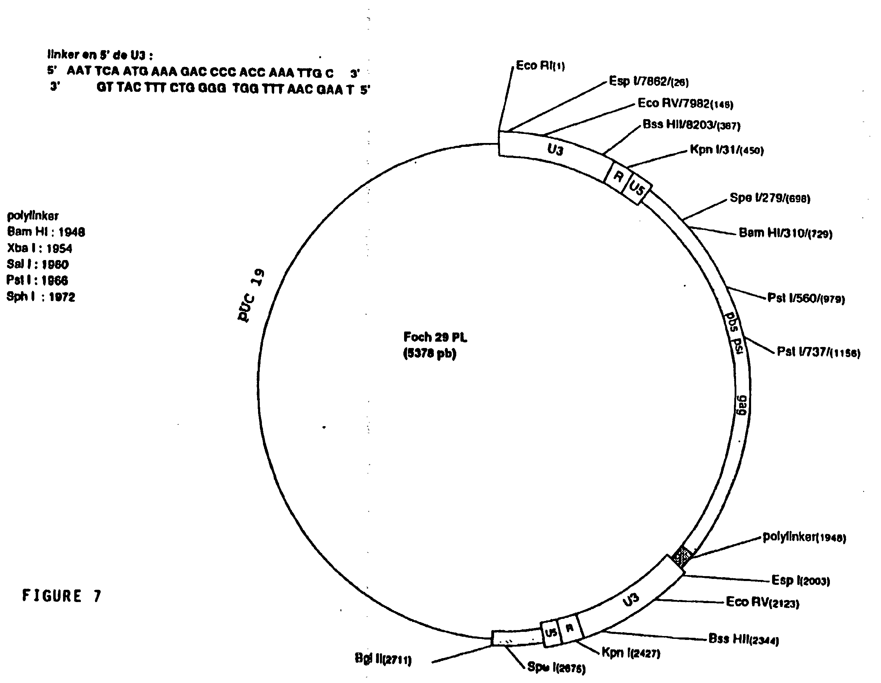

Un vecteur comportant le fragment ClaI-BamHI contient donc deux séquences LTR, LTR5' et LTR3' ayant la même origine virale. Ce vecteur peut être modifié par suppression de tout ou partie de la séquence virale d'enveloppe présente en amont et/ou en aval des séquences LTR5' et LTR3'. Un vecteur de ce type est par exemple pFOCH29-PL décrit à la figure 7.

Ces séquences LTR peuvent être séparées sur le vecteur par la présence de la séquence gag dont il a été question ci-dessus et/ou par la séquence de nucléotides exogène que l'on cherche à transférer, cloner ou exprimer.

Selon un mode de réalisation intéressant de l'invention, le vecteur recombinant est caractérisé en ce qu'il comprend la totalité du fragment ClaI-PuvII, comprenant les nucléotides 7702 à 1527 de la séquence représentée à la figure 1.

Un autre vecteur rétroviral préféré comprend la totalité du fragment ClaI-BamHI (7702 à 310).

Un vecteur rétroviral de l'invention est utilisable à des fins thérapeutiques ou diagnostiques dans le but d'introduire chez un patient, une séquence de nucléotides d'intérêt clinique. Le vecteur de l'invention présente en effet les qualités d'efficacité et de sécurité requises pour cette application.

Avantageusement le contrôle du clonage, de l'expression ou du transfert de la séquence exogène est réalisé selon l'invention, quelle que soit l'orientation transcriptionnelle de cette séquence par rapport à l'orientation transcriptionnelle du virus.

Une séquence de nucléotides exogène selon l'invention, est une séquence de nucléotides qui n'est pas naturellement contenue dans le matériel génétique constituant le vecteur, et en particulier dans les séquences nécessaires au contrôle de l'expression du clonage ou du transfert. Il peut s'agir d'une séquence naturelle ou synthétique, notamment d'une séquence hybride.

Par l'expression "transfert d'une séquence de nucléotides exogène", on entend l'incorporation d'une séquence portée par le vecteur, dans le génome ou satellite de celui-ci, d'une cellule transformée par ce vecteur. Un tel transfert peut être le résultat d'une recombinaison, en particulier d'une recombinaison homologue.

Le vecteur de l'invention peut donc permettre l'expression définitive dans le génome d'une cellule cible d'une séquence de nucléotides exogène choisie du fait de sa propriété d'intégration au génome des cellules cibles.

Selon un mode de réalisation avantageux de l'invention, la séquence exogène et la séquence contenue dans le fragment ClaI-PvuII ou dans le fragment ClaI-BamHI, ou l'un de ces fragments selon la description ci-dessus, sont insérées dans un plasmide, par exemple dans le plasmide Puc19, le plasmide Puc18, ou tout autre plasmide approprié.

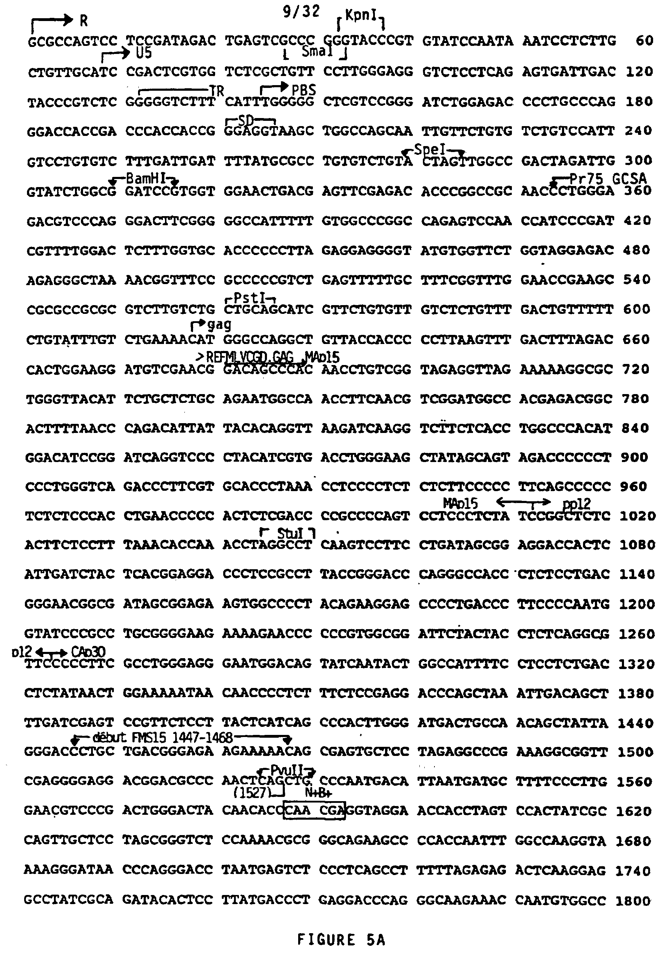

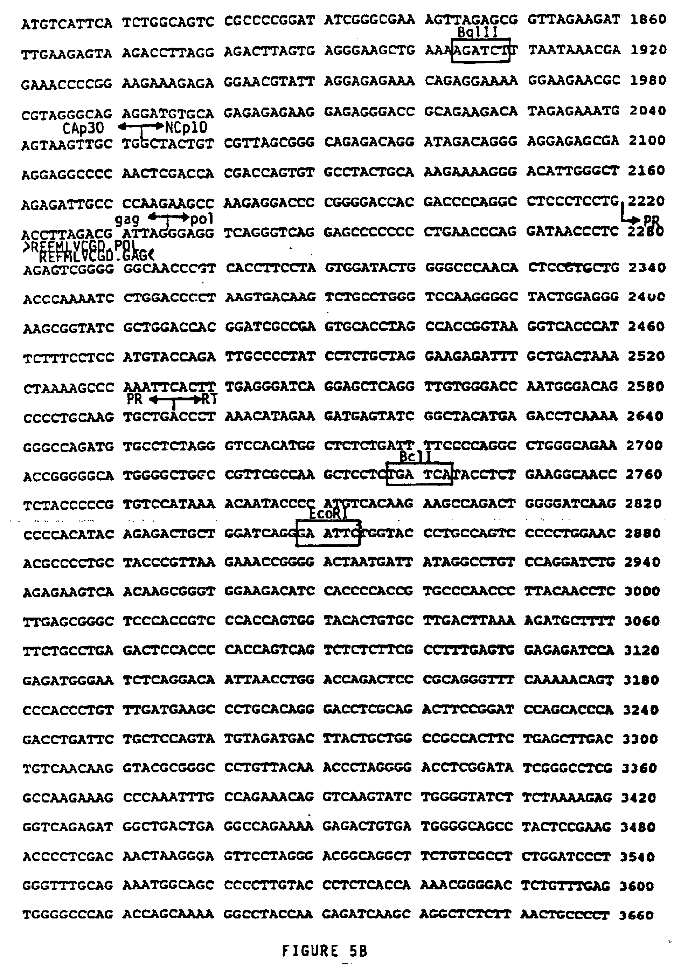

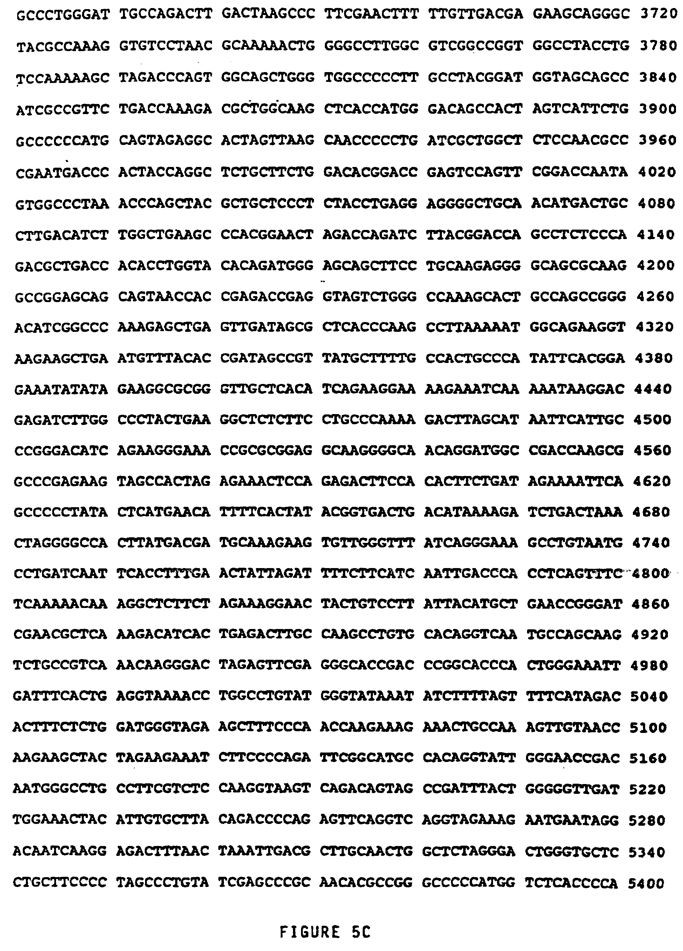

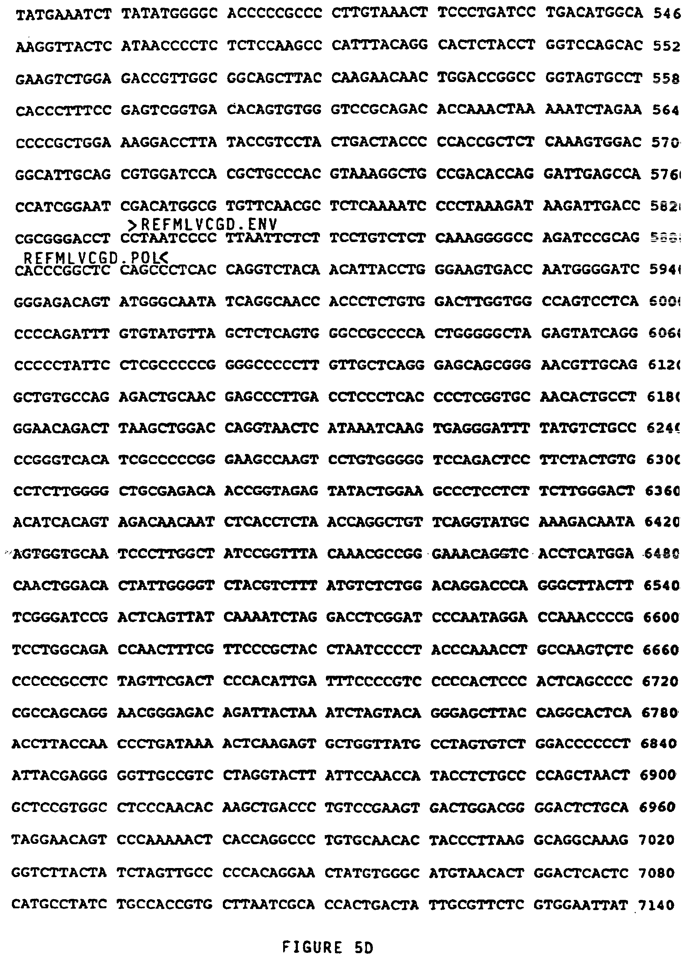

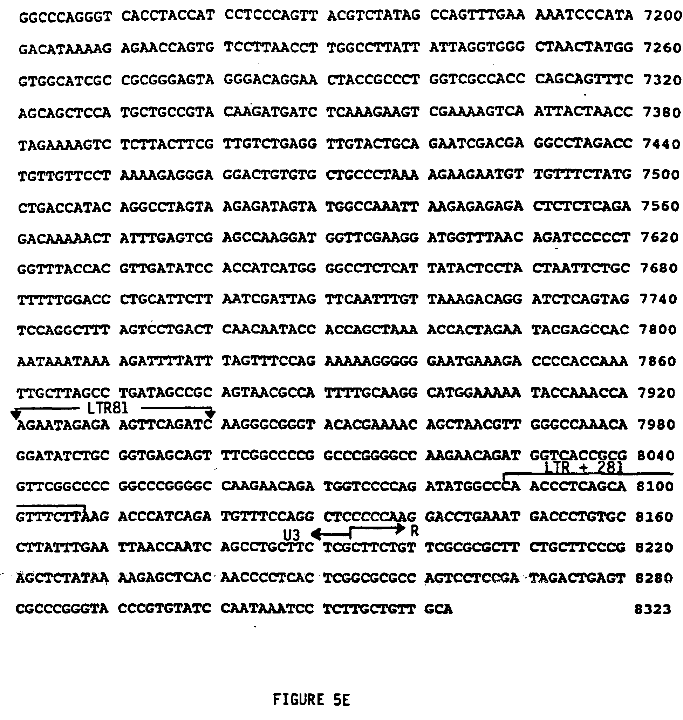

De préférence, le vecteur recombinant comprend en outre une partie de la séquence gag située entre les nucléotides 619 et 2245 de la séquence de la figure 5, en particulier la séquence comprise entre les nucléotides 619 et 1527 de la séquence de la figure 1.

La présence d'un fragment ou de la totalité de la séquence gag peut contribuer à la stabilisation du vecteur obtenu. La séquence gag code pour les nucléoprotéines du virus de Friend et augmente l'efficacité d'encapsidation tout au moins dans sa partie proximale.

Toutefois, on peut avoir intérêt à limiter la partie de la séquence gag présente dans le vecteur de l'invention en fonction de la taille de la séquence exogène introduite dans le vecteur afin d'obtenir un titre viral infectieux plus élevé et diminuer la production de protéines virales et le risque de génération de virus compétents pour la réplication. De préférence, la partie de la séquence gag présente dans le vecteur sera inférieure à environ 2/3 de la séquence normale gag. Avantageusement, la séquence conservée de gag est la partie de cette séquence intervenant dans l'étape d'encapsidation du vecteur rétroviral obtenu.

Un vecteur intéressant de l'invention est caractérisé en ce qu'il est essentiellement dépourvu des séquences virales pol et/ou env.

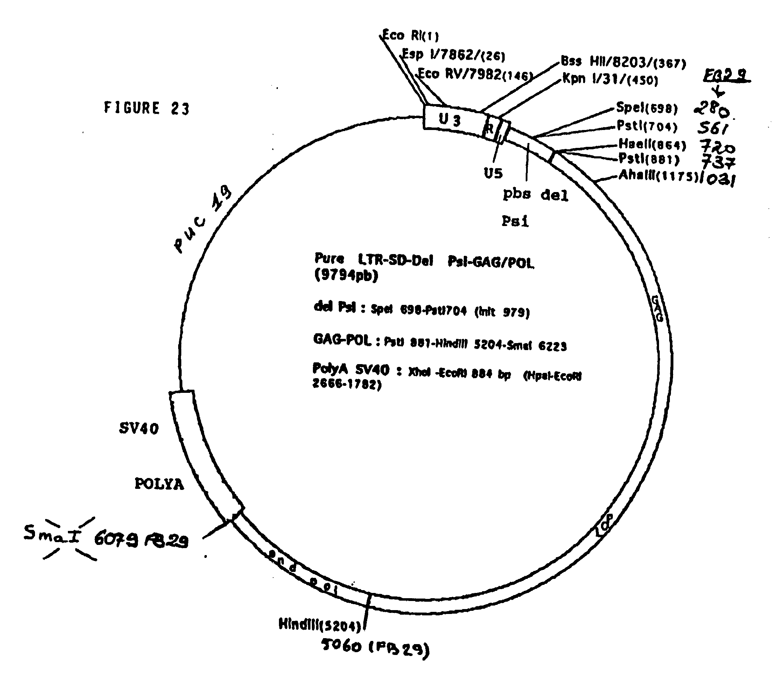

En revanche, les séquences pol et gag de la souche FB29 telles que représentées à la figure 5, peuvent être conservées en aval du LTR pour la réalisation de lignées d'encapsidation (voir à titre d'exemple figure 23).

L'expression des séquences pol et gag peut être contrôlée par un promoteur distinct du LTR viral qui est alors supprimé.

L'invention a donc pour objet des constructions telles que décrites ci-dessus comportant une séquence contenue dans le fragment ClaI-PvuII précité et comprenant en outre les séquences gag et pol ou une partie de ces séquences, suffisante, pour la réalisation de lignées d'encapsidation.

Ces lignées peuvent être utilisées pour encapsider le vecteur rétroviral de l'invention.

Un vecteur comprenant à la fois le fragment ClaI-PvuII (qui porte une unique séquence LTR) et les séquences gag et pol ou une partie de ces séquences peut être en particulier mis en oeuvre dans le cadre de la préparation de lignées d'encapsidation pour le transfert de gènes ex vivo ou in vivo, ledit gène étant représenté par la séquence de nucléotides exogène.

Lorsqu'une partie de la séquence virale env est présente dans le vecteur, elle reste insuffisante pour permettre des recombinaisons susceptibles de produire des virus compétents pour le réplication.

Un vecteur avantageux de l'invention dépourvu de séquence d'enveloppe comprend le fragment comportant les nucléotides 7806 à 1527 de la séquence représentée à la figure 1.

L'invention concerne aussi un vecteur recombinant tel que la séquence contenue dans le fragment ClaI-PvuII (7702-1527) et/ou ce fragment, et/ou la séquence contenue dans le fragment ClaI-BamHI (7702-310) et/ou ce fragment est remplacée soit par une séquence hybridant dans des conditions de forte stringence avec la séquence correspondante des susdits fragments, soit par une séquence ayant une homologie d'au moins 95% en nucléotides avec la séquence correspondante des susdits fragments ou d'au moins 85% s'agissant de la séquence U3.

L'hybridation est réalisée dans les mêmes milieux d'hybridation que ceux décrits à la partie expérimentale en ajoutant toutefois, deux rinçages pendant 10 min. à 65°C dans un milieu SSC lx, SDS 0,1 ainsi que 2 rinçages pendant 10 min. à 65°C dans un milieu SSC 0,1x, SDS 0,1.

La nomenclature des nucléotides est donnée ci-dessus par référence à la numérotation des nucléotides de la séquence virale dans la présentation donnée à la figure 1.

Le cas échéant, l'une des deux séquences LTR définies précédemment, à partir de la séquence du virus F-MuLV (souche FB29 du virus de Friend) peut être remplacée par une séquence LTR provenant d'un autre virus, par exemple du virus de la leucémie murine de Moloney (Mo-MuLV).

De même le vecteur recombinant de l'invention peut également contenir d'autres séquences rétrovirales que celles qui ont été décrites ci-dessus, soit issues du même virus F-MuLV dont la séquence est donnée à la figure 5, soit issues d'un autre virus.

L'invention a également pour objet un vecteur recombinant caractérisé en ce qu'il contient en outre au moins un adaptateur multiple présentant des sites de restriction uniques par rapport aux sites contenus dans le vecteur.

Un tel adaptateur, de préférence à sites multiples (polylinker) a pour but de permettre notamment l'insertion d'une ou plusieurs séquences exogènes dont on recherche le transfert, le clonage ou l'expression.

Un vecteur particulièrement intéressant est le vecteur caractérisé en ce qu'il s'agit du plasmide pFOCH29 déposé sous la dénomination pFOCH29-SCS1 dans une souche de E.coli SCS1 à la C.N.C.M. le 30 Juin 1993 sous le n°I-1326.

La souche E.coli SCS1 est commercialisée par la société STRATAGENE Corp.

Un vecteur de l'invention peut également comprendre un gène ou une partie d'un gène marqueur comme par exemple le gène de résistance à la néomycine. La présence d'un gène marqueur facilite notamment la détection de la présence du vecteur dans des cellules recombinantes.

L'invention a également pour objet un vecteur recombinant répondant aux définitions précédentes, dans lequel la région U3 du LTR est délétée au moins en partie, de façon telle que les séquences transcriptionnelles, notamment le promoteur et/ou l'activateur contenu(s) dans U3 est (sont) au moins en partie inactivé(s) ou modifié(s).

Dans ce cas, on est en présence d'un vecteur capable de s'autoactiver ou vecteur SIN ("self inactivating vector"). Un vecteur SIN ainsi construit permet l'expression de la séquence exogène qu'il contient, lorsque celle-ci est placée sous le contrôle d'un promoteur dit "promoteur interne", de nature virale ou non le cas échéant le promoteur de cette séquence, ou un promoteur tel que le promoteur du récepteur à l'EGF (Epidermal Growth Factor) ou le promoteur ubiquitaire PGK de la phosphoglycérate kinase.

L'avantage consiste à améliorer la sécurité (non propagation et diminution du risque d'activation des séquences du voisinage). Un autre avantage est de tirer partie de l'intégration médiée par les rétrovirus mais de spécifier ou de cibler la transcription par le promoteur interne.

De même la séquence U5, voire le cas échéant la séquence R, peut être délétée au moins en partie.

Cette délétion peut être réalisée au niveau d'un unique enchaînement LTR présent dans le vecteur ou éventuellement au niveau de chaque séquence LTR de ce vecteur.

Elle conduit cependant le plus souvent à une diminution du titre en particules virales voire à l'absence d'intégration dans le génome de la cellule cible (si U5 délété).

Selon un autre mode de réalisation de l'invention, la séquence de nucléotides exogène est sous le contrôle d'un promoteur exogène (ou interne).

Par "promoteur exogène", on entend un promoteur qui n'est pas naturellement présent dans le vecteur. Un tel promoteur peut être le promoteur naturel de la séquence exogène. Il peut s'agir d'un promoteur constitutif ou d'un promoteur inductible.

Un vecteur recombinant défini précédemment est de préférence introduit par exemple par transfection ou électroporation, dans une lignée d'encapsidation. Cette transfection permet la constitution de particules virales, propres à la réalisation de recombinaisons par transduction dans des cellules cibles en vue du clonage, du transfert ou de l'expression de la séquence de nucléotides exogène contenue dans le vecteur.

Ainsi, un vecteur particulièrement intéressant peut être transfecté dans une lignée psi-CRIP.

On peut également avoir recours à la lignée d'encapsidation psi-CRE ou à toute autre lignée dès lors qu'elle ne conduit pas à des recombinaisons susceptibles de donner lieu à la production de particules virales de type sauvage à partir de l'ADN proviral contenu dans le vecteur.

Selon un autre mode de réalisation de l'invention, le vecteur recombinant peut être introduit dans des liposomes ou dans un complexe macromoléculaire (Monsigny M. et al M/S 1993).

Le vecteur FMuMV peut être utilisé pour réaliser de telles lignées d'encapsidation, selon la technique illustrée dans McLachlin JR et al, 1990. Une telle lignée peut être construite à partir des séquences des gènes gag, env et pol, la séquence d'encapsidation étant délétée et une au moins des séquences gag, pol ou env comportant une mutation ponctuelle qui n'altère pas la protéine résultante.

Les vecteurs de l'invention peuvent contenir une ou plusieurs séquences exogènes. Ces séquences peuvent être insérées en dehors des fragments ClaI-BamHI ou BamHI-BamHI comme on l'a vu plus haut, ou au contraire peuvent être insérées au sein de ces fragments et en particulier dans leur séquence LTR.

Ces vecteurs peuvent aussi contenir des éléments de ciblage cellulaire pour orienter l'intégration du vecteur dans des cellules déterminées.

Avantageusement, les constructions de vecteur rétroviral selon l'invention permettent lors de l'infection des cellules cibles, l'obtention d'un titre viral égal ou supérieur à 10⁴ pfu/ml évalué lorsque la séquence de nucléotides exogène codant pour le gène de la néomycine est insérée dans ce vecteur.

Les applications de ce vecteur peuvent être de nature très diverse et en particulier ces vecteurs peuvent être utilisés pour le clonage, l'expression et/ou le transfert de séquences de nucléotides ayant un intérêt clinique (thérapeutique ou diagnostique).

Ainsi on peut utiliser les vecteurs de l'invention pour le transfert dans des cellules par exemple des cellules somatiques, de gènes à visée thérapeutique quelle(s) que soi(en)t la ou les maladie(s) ou pathologie(s) concernée(s).

Les séquences d'intérêt thérapeutique dont il est question ici sont par exemple des séquences correspondant à l'équivalent normal d'un gène non fonctionnel dans le cas d'une pathologie donnée, ou encore une séquence antisens ou un mutant négatif dominant d'un gène donné ou une séquence codant pour un inhibiteur fonctionnel d'un gène, ou l'utilisation d'un gène marqueur.

Le vecteur de l'invention est ainsi approprié pour la thérapie génique du cancer par application des techniques de correction génique ou d'amélioration des stratégies de destruction des cellules tumorales. Selon la première technique, on peut utiliser le vecteur pour corriger des mutations constitutionnelles dans le cas de la prédisposition héréditaire au cancer, des anomalies de la transduction du signal, comme les voies médiées par l'oncogène ras et ses homologues, des anomalies activatrices d'oncogènes, des anomalies inhibitrices de gènes suppresseurs de tumeurs, des anomalies favorisant l'instabilité génétique, des anomalies portant sur la réparation de l'ADN.

Selon la deuxième technique, le vecteur peut être mis en oeuvre pour activer des prodrogues comme dans le cas du gène thymidine kinase du virus Herpès transformant le Ganciclovir ou l'Acyclovir en drogues cytotoxiques, ou le gène de Cytosine déaminase transformant un précurseur du 5-fluorouracile en drogue active, ou pour induire ou stimuler le système immunitaire par manipulation des cellules tumorales, avec par exemple des gènes de cytokines, par manipulation des cellules présentatrices de l'antigène ou de leurs précurseurs (souches hématopoiétiques) ou par manipulation des cellules effectrices de l'immunité, cellules T, cellules B, LAK, TILs.

S'agissant de la correction de maladies génétiques, d'anémies, l'invention est par exemple applicable à la correction d'erreurs innées du métabolisme, de maladies de l'hémoglobine, comme les thalassémies ou la drépanocytose, de maladies de l'hémostase et de la coagulation, de maladies héréditaires de la démyélinisation ou de myopathies.

Les vecteurs de l'invention sont également appropriés pour vacciner des patients contre des agents pathologiques, et ce de façon permanente ou transitoire.

Le transfert peut être réalisé par transduction dans des cellules, tissus, organes ou organismes.

Selon un autre mode de réalisation de l'invention, la séquence de nucléotides exogène code pour un antigène ou un déterminant antigénique.

Un vecteur contenant un tel déterminant antigénique pourra être utilisé à titre de vaccin permanent ou transitoire ou le cas échéant dans le cadre d'un protocole thérapeutique, par exemple pour susciter une réponse immunitaire.

A titre d'exemple, des séquences des antigènes des rétrovirus HIV peuvent être incorporées dans le vecteur de l'invention.

A cet égard, le vecteur rétroviral de l'invention peut être appliqué à l'immunisation intra-cellulaire en utilisant des mutants transdominants de CD4, Tat, Rev, Gp120, des leurres avec hypersynthèse de protéines régulatrices comme TAR, des ribozymes spécifiques de séquences virales ou des gènes antisens.

Cette même technique peut être utilisée pour traiter d'autres infections rétrovirales.

La séquence de nucléotides exogène dont il est question précédemment peut être une séquence d'ADN génomique ou une séquence d'ADNc ou encore une séquence d'ARN.

De même, cette séquence peut être naturelle ou synthétique.

L'invention a également pour objet une cellule recombinante procaryote ou eucaryote caractérisée en ce qu'elle est modifiée par un vecteur recombinant de l'invention. Avantageusement il s'agit d'une cellule d'une espèce ne comportant pas de rétrovirus endogène.

Une telle cellule est avantageusement une cellule de mammifère, en particulier une cellule humaine.

De même il s'agit soit d'une cellule totalement différenciée ou d'une cellule de type précurseur. Par exemple le vecteur de l'invention est particulièrement approprié pour la modification de cellules hématopoïétiques et également de précurseurs de cellules hématopoïétiques, ou d'une cellule d'une souche totipotente lymphomyéloide.

Par ailleurs, des cellules nerveuses, neuronales ou gliales, peuvent également être modifiées par le vecteur de l'invention.

D'autres cellules peuvent être modifiées par les vecteurs de l'invention par exemple des lymphocytes T ou B, ou autres médiateurs de l'immunité cellulaire, des cellules tumorales, du stroma médullaire, des cellules endothéliales ou mésenchymateuses.

Le vecteur recombinant selon l'invention peut aussi être mis en oeuvre pour modifier des fibroblastes, des cellules cutanées, des cellules hépatiques ou des cellules musculaires.

D'autres cibles cellulaires peuvent être transformées par le vecteur de l'invention. On citera par exemple les cellules épithéliales, par exemple de l'épithélium vésical ou mammaire, les cellules tumorales, les cellules accessoires du système nerveux telles que les précurseurs d'oligodendrocytes ou les cellules de Schwann.

On peut aussi modifier des lignées cellulaires comme les lignées de lymphocytes T JURKATT, les lignées de cellules NK telles que YT2C2 les lignées de monocytes-macrophages (par exemple U937) ou les lignées erythro-megacaryocytaires (par exemple K562).

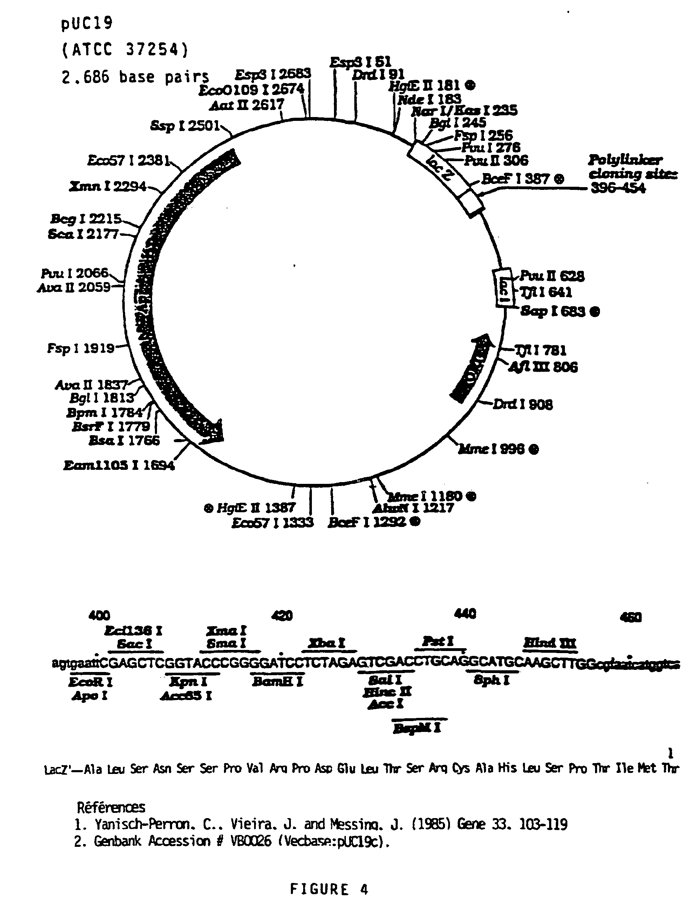

D'autres caractéristiques et avantages de l'invention apparaissent dans les exemples et les figures qui suivent.
Figure 1 : Séquence de l'ADN viral utilisé pour la réalisation du vecteur FOCH29.

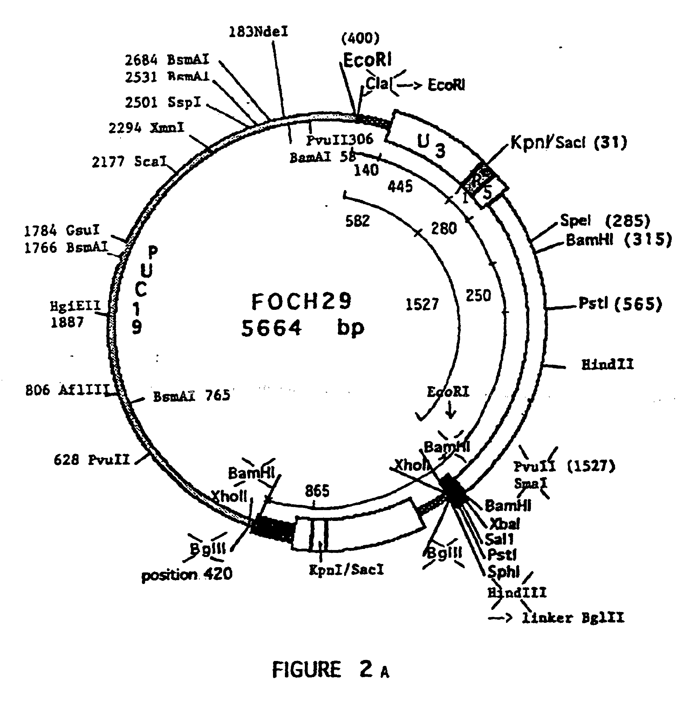

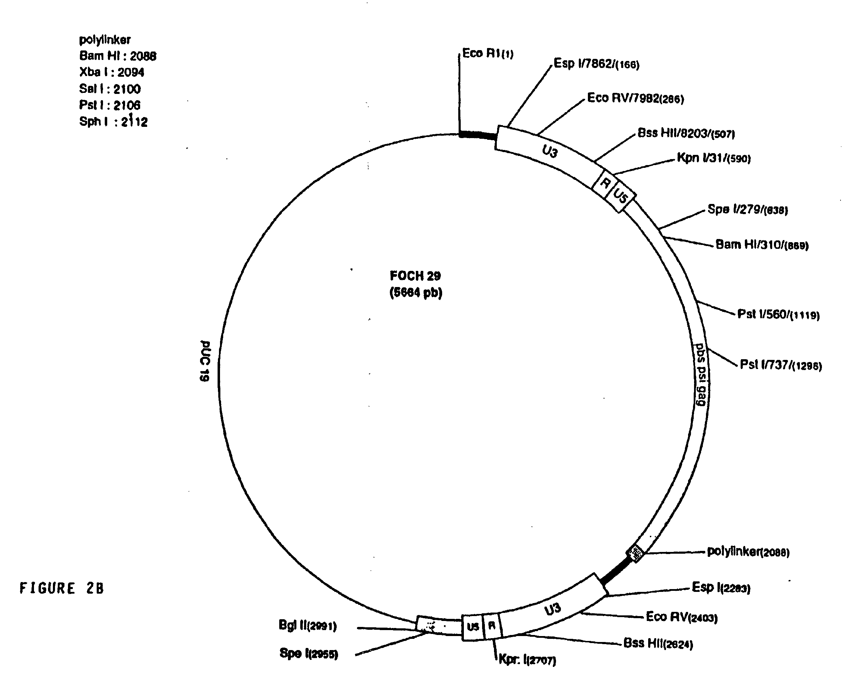

Figure 2 : A: Carte de restriction du vecteur FOCH29. Côtes Seq "FB29"
   Cla --> U3 140 (7702-7845)
   U3 --> R 410 (7845-8255)
   R --> CBS 145 (0-145)
   PvuII --> BamHI 208
   PvuII --> PvuII 1098
   PvuIIMT --> PvuII 1669
   BsmAI --> 55/150/765/1766/2531/2684
   B: Carte de restriction de FOCH 29 dans laquelle les sites sont indiqués avec la numérotation de la séquence FB 29 de la figure 1 et avec la numérotation propre à la construction ainsi réalisée (numéros entre parenthèses).

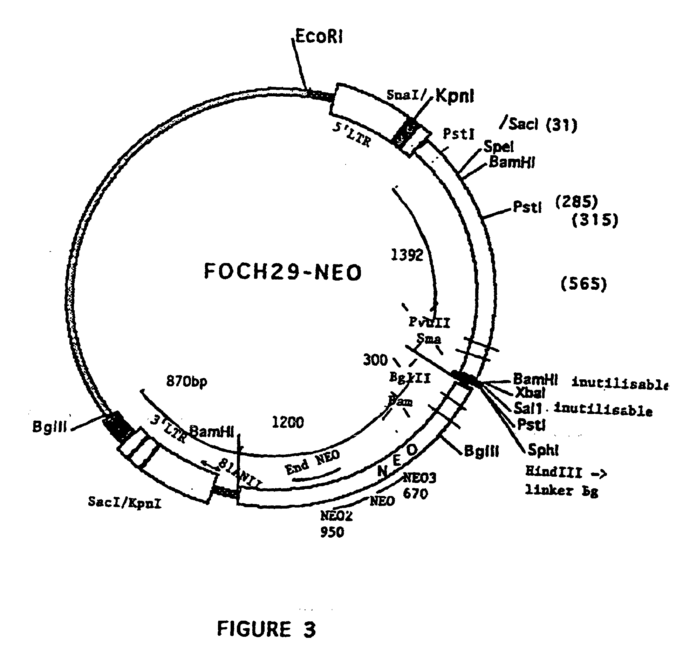

Figure 3 : Carte de restriction du vecteur FOCH29-Neo. Le gène néo a été cloné dans le polylinker. Les enzymes utilisables dans le polylinker sont Xba, Sal, SphI.
   Coupures:
   - BglII 2070
      5300
   - Bam 1392
      1500
      4456
   - Sal/BglII 300
      2070
      5000
Figure 4 : Carte de restriction du plasmide pUC19.
Figure 5 : Séquence du FMuLV.

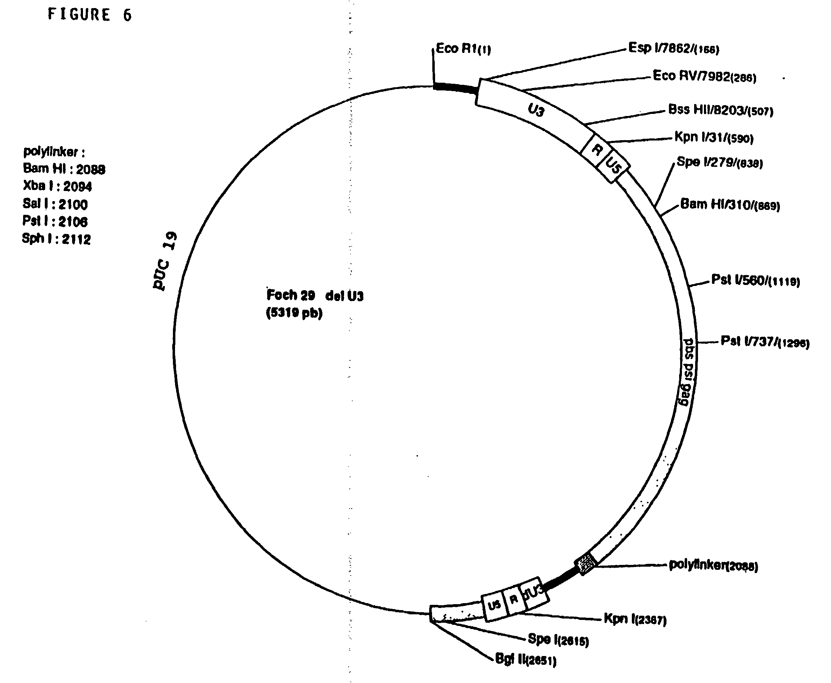

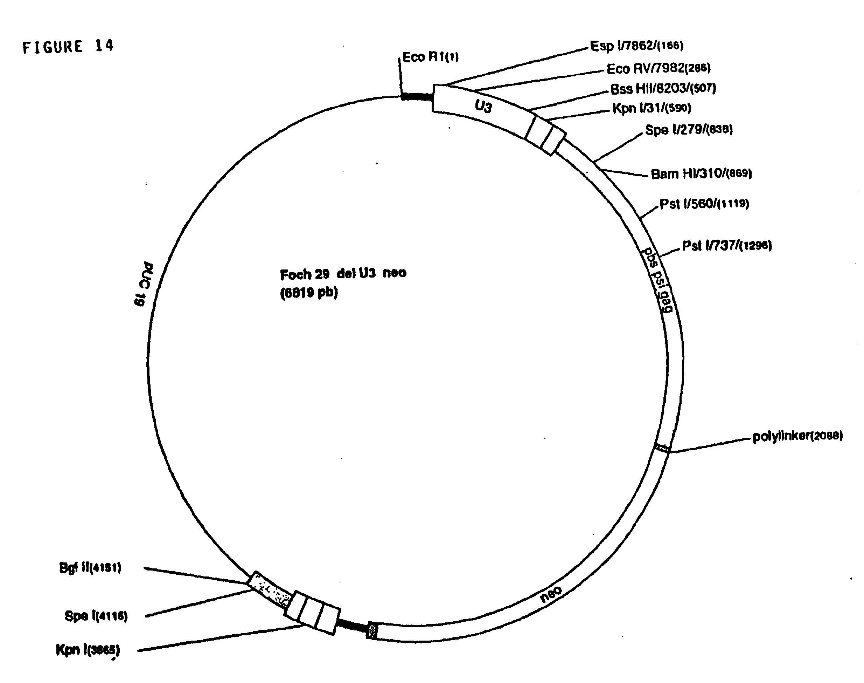

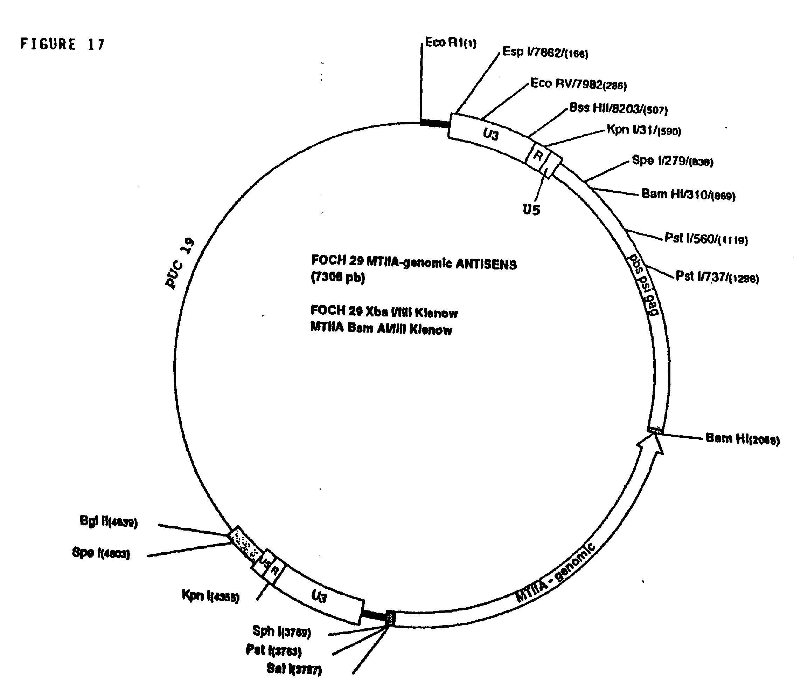

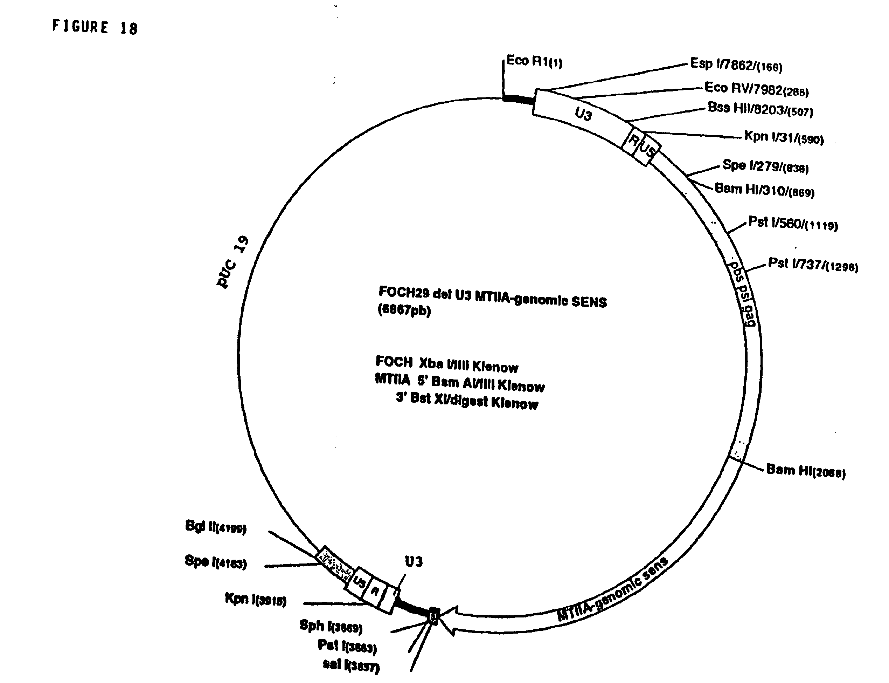

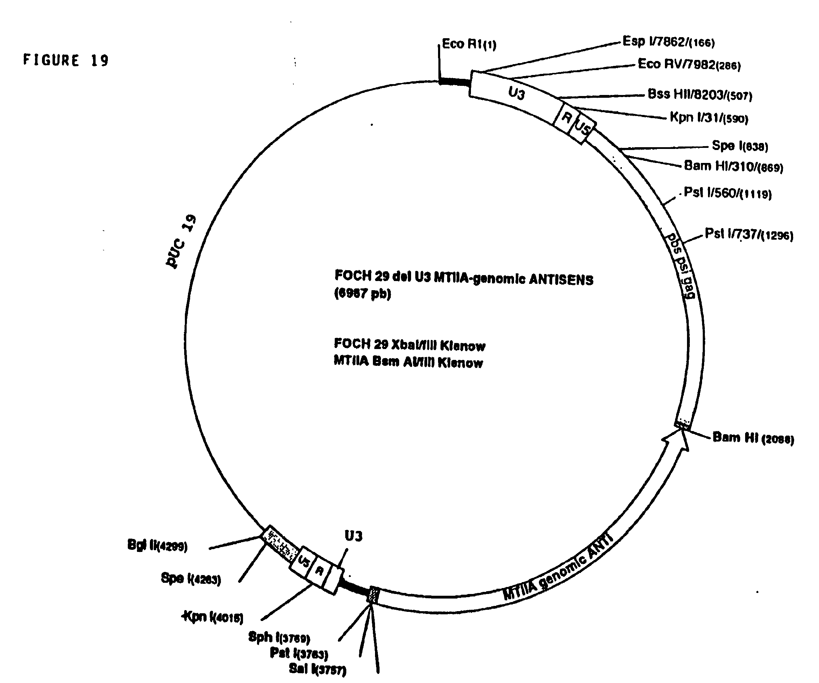

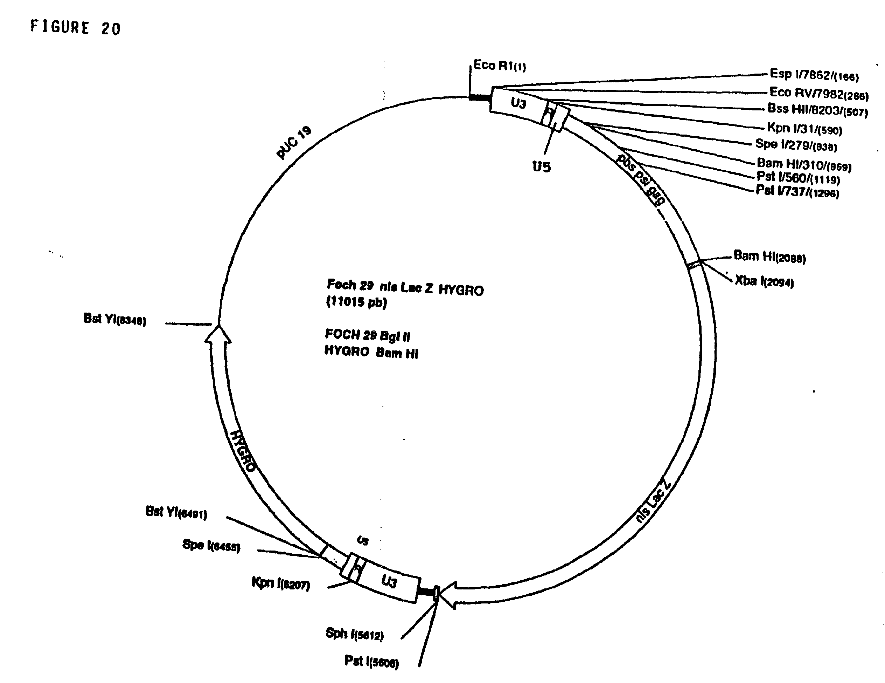

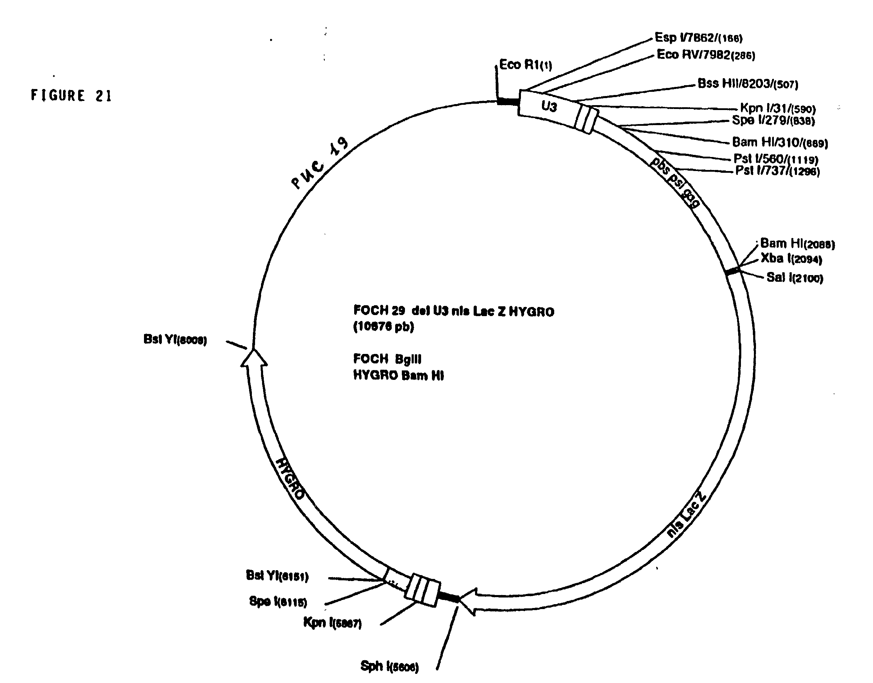

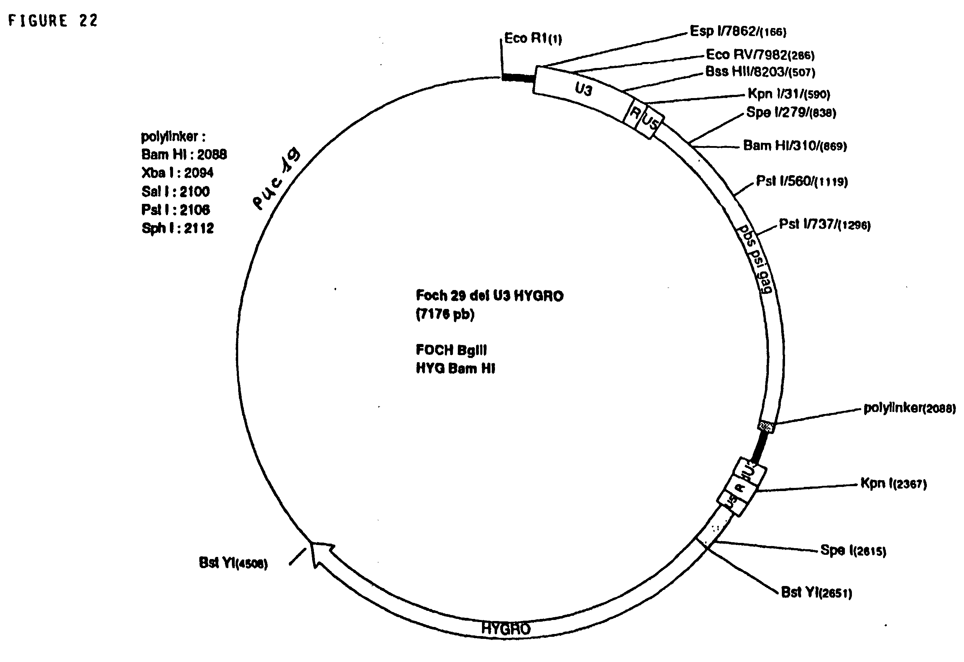

Figures 6 à 24 : cartes de restriction des constructions réalisées à partir du vecteur rétroviral FOCH29.

### Exemples

### A) PREPARATION DU VECTEUR RETROVIRAL

### MATERIEL ET METHODES

### 1- Source du matériel génomique viral

L'ADN génomique du provirus a été cloné dans pBR322 (Sitbon et al., 1986). Après remplacement du site ClaI en 7702 de la séquence du virus par un site EcoRI, le fragment EcoRI- PvuII de 2110 paires de bases (pb) contenant l'intégralité du Long Terminal Repeat viral (LTR) a été sous-cloné aux sites EcoRI et SmaI du polylinker (adaptateur multiple) de pUC19.

### 2- Construction du vecteur rétro-viral FOCH29

Le site HindIII du polylinker de pUC19 contenant le fragment EcoRI-PvuII a été remplacé par un site BgIII, après ouverture par HindIII, remplissage par le fragment long de l'ADN polymèrase d'E.Coli (fragment Klenow) et ligation avec un adaptateur BqlII ne recréant pas le site HindIII ; le site BglII a été introduit pour recevoir un fragment BamHI-BamHI de 865pb contenant un second exemplaire du LTR natif du virus de Friend destiné à constituer le LTR d'aval (ou 3'LTR). Ce fragment BamHI-BamHI a été isolé en remplaçant le site EcoRI de pUC19 par un site BamHI d'amont au moyen d'un adaptateur (linker) ne recréant pas le site EcoRI après remplissage des extrêmités par le fragment Klenow de l'ADN polymèrase ; le site BamHI d'aval est endogène à la séquence virale.

Ce fragment a donc été introduit par ligation avec l'armature de base (pUC19) dont l'ouverture par BglII permettait de ménager des extrémités cohésives avec les extrémités générées par BamHI. Le plasmide résultant est appelé pFOCH29.

### 3- Introduction d'un gène marqueur

Le fragment d'ADNc BglII-BamHI (1500 pb) du gène de résistance à la Néomycine dérivé du rétrotransposon Tn5 (NéoR) a été introduit entre les deux LTRs viraux après ligation des trois fragments : pUC19-5'LTR BglII, NéoR BglII-BamHI et 3'LTR BamHI-BamHI. Le plasmide résultant est appelé pFOCH29-Néo.

### 4- Transfection de lignées d'encapsidation Psi-CRIP et infection de fibroblastes

Le plasmide pFOCH29-Néo a été introduit dans la lignée d'encasidation amphotrope psi-CRIP décrite par Danos et al. (1988) par transfection utilisant la précipitation au phosphate de calcium selon la procédure standard, sans ADN carrier ; 10 microgrammes de plasmide ont été déposés sur une boite de culture de 35mm de diamètre où 5.10⁴ cellules ont été plantées la veille.

Les cellules psi-Crip ont été cultivées en milieu de Dulbecco modifié Eagle (DMEM, Gibco-BRL) additionné de 10% de sérum de veau nouveau-né (HyClone). Deux jours après la transfection les cellules ont été trypsinées, diluées au 1/20 ème et mises sous sélection, en présence de Généticine à la concentration finale de 1 milligramme (mg) par Millilitre (ml) de milieu de culture. Les colonies apparues après 12 jours ont été prélevées et réimplantées sur des boites de culture de 24 puits, à raison d'un clone par puits.

Le surnageant de culture de cellules d'un puits parvenu à confluence a été prélevé, filtré sur 0,45 µm pour éliminer les cellules en suspension et utilisé pour infecter des fibroblastes murins (NIH3T3) plantés de façon identique sur des boites de culture de 24 puits, en présence de polybrène à la concentration de 8 *µ*g/ml. Les NIH3T3 ont été cultivées en DMEM additionné de 10% de sérum de veau foetal (SVF). L'intégration virale a été analysée par PCR sur un lysat de NIH3T3 parvenues à confluence.

### 5- Réaction en chaîne de la Polymérase (Polymerase, Chain Reaction ou PCR)

Le surnageant du lysat des NIH3T3 confluentes sur un puits de boîte de culture de 24 puits a été recueilli dans 100 *µ*l dont 10 ul ont été utilisés dans la réaction de PCR, qui est pratiquée dans le tampon suivant : tampon de PCR 10X standard incluant 25mM de MgCl₂ (Perkin-Elmer/Roche MS) ; 100 nanogrammes (ng) de chaque amorce ; 2ul de dNTPs 10mM (mélange équimolaire de chaque dNTP à la concentration initiale de 10 mM, soit 2,5mM chacun) ; 2 unités de Taq Polymèrase clonée (Perkin-Elmer/Roche MS) pour 40 cycles, une seule unité pour 25 cycles ; pour un volume final de 50*µ*l.

Deux couples d'amorces ont été utilisés.

Les séquences des oligonucléotides utilisés sont :
1°) - pour le premier couple : 5'CTGCTGACGGGAGAAGAAAAAC-3' / 5'CCCGCTCAGAAGAACTCGTC-3' ;
2°) - pour le second couple : 5'GACGAGTTCTTCTGAGCGGG-3' / 5'GATCTGAACTTCTCTATTCTTG-3'

La taille des séquences amplifiées est pour le premier couple, Fin-gag/deux tiers proximaux gène NéoR : 900 pb ; et pour le second couple, tiers distal gène NéoR/ moitié proximale du LTR 3': 610 pb.

Dénaturation 5 min à 94°C ; 40 cycles sur GeneAmpPCR 9600 avec dénaturation 30" à 94°C ; annealing 15" à 55°C et élongation 30" à 72°C ; suivis d'une étape terminale d'élongation de 10 min.

Les échantillons (15ul sur 50ul) ont été déposés sur gel d'agarose à 1,2% (Seakem, FMC) et ont été soumis à une électrophorèse horizontale de 45 min à 80 volts ; la détection du signal reposant sur l'analyse de l'intensité de fluorescence en bromure d'éthidium (BET).

### 6- Détermination des titres infectieux

Chacun des clones ayant été testé pour sa capacité à infecter des NIH3T3 a été amplifié et éventuellement congelé dans l'attente de l'analyse par PCR de l'efficacité d'infection.

Après PCR deux clones principaux ont été retenus et amplifiés afin d'infecter des NIH3T3 selon une procédure standard.

1ml de surnageant de culture de 16 heures sur une boite de 35mm de diamètre de chaque clone producteur a été prélevé à confluence, filtré sur 0,45um afin d'éliminer les cellules productrices en suspension. Le surnageant a été mis en contact avec les cellules NIH3T3 à 50% de confluence sur une boite de culture de même diamètre (35mm), en présence de polybrène à la concentration de 8ug/ml de milieu. Les cellules ont été incubées pendant 2H30 environ, à 37°C ; une agitation a été pratiquée chaque demi-heure. Trois volumes de milieu frais ont été ajoutés après 2H30.

### TITRES INFECTIEUX VIRAUX

Des dilutions successives du surnageant primaire ont été utilisées pour infecter des cellules NIH3T3 ; surnageant pur, dilué au 1/10ème, au 1/1000ème et au 1/100 000ème. Deux jours après l'infection, les cellules ont été trypsinées passées au 1/20ème environ sur trois boites de culture de 100 mm de diamètre et mises sous sélection par addition de Généticine (1mg/ml) au surnageant.

Cette procédure expérimentale a été rendue plus stringente dans la mesure où : d'une part, la drogue est ajoutée très vite après l'infection ; et d'autre part, la mise en sélection directe sans trypsiner les cellules ne permet pas de multiplier artificiellement le nombre de colonies résultantes, les cellules filles issues d'une cellule infectée restant groupées et ne formant qu'une colonie unique in situ. A l'inverse, lorsque les cellules sont trypsinées, les cellules filles essaiment dans la boite de culture réceptrice et forment des colonies artificiellement indépendantes qui, si elles sont dénombrées, multiplient artificiellement le titre.

### SOUTHERN BLOT

Deux jours après l'infection par du surnageant pur, les NIH3T3 ont été trypsinées, passées au 1/20ème environ sur trois boîtes de culture de 100 mm de diamètre, dont l'une est mise sous sélection par la généticinè.

A confluence, l'ADN génomique des cellules infectées par chacun des deux clones après ou sans sélection, est extrait puis quantifié.

L'ADN a été digéré par deux enzymes de restriction, PstI et KpnI afin de réaliser un Southern blot. Après contrôle de la qualité de la digestion et homogénéisation de la quantité d'ADN déposée dans chaque puits, le transfert a été réalisé sur une membrane de Nylon Hybond N (Amersham). L'hybridation a été conduite avec une sonde incluant la totalité des séquences du LTR viral encadrées par 100 bases en amont et 100 bases en aval. La sonde a été marquée par extension d'amorce (Feinberg et Vogelstein, 1983, 1984) avec du dCTP marqué à l'alpha-32-P, à une activité spécifique de 5.10⁸ cpm/µg.

L'hybridation a été pratiquée dans un milieu constitué de : 50% formamide désionisée ; 5X SSEP ; 1X denhardt's ; 5% dextran sulfate et 0,2mg/ml d'ADN de saumon soniqué, pendant 20 heures à 42°C. De brefs rinçages ont été pratiqués dans une solution peu stringente 2X SSEP/0,1% SDS, 5min à température ambiante et 10 min à 65°C ; suivis d'une exposition de 3 jours sur films Kodak-XAR-5 à -80°C avec écrans amplificateurs Li-Plus (Dupont-NEN).

### 7- Recherche de production de virus Helper

Cette recherche a été effectuée par test de mobilisation sur lignées 3T3BAG (Danos et al, 1988 ; Danos, 1991).

Les cellules 3T3BAG ont dans un premier temps été infectées par le surnageant pur de lignées d'encapsidation infectées. Plusieurs cycles successifs d'infection de 3T3BAG vierges avec le surnageant des 3T3BAG précédemment infectées ont été pratiqués pour sensibiliser le test.

### RESULTATS

### 1- Construction du vecteur rétro-viral FOCH29

La souche virale FB29 du virus de la leucémie murine de Friend a été isolée (Mathieu-Mahul et al., 1982) et l'ADN génomique du provirus intégré a été cloné dans pBR322 (Sitbon et al;, 1986). Cet ADN génomique a été entièrement séquencé (Perryman et al., 1991). Le fragment génomique ClaI-PvuII de 2120 pb a été sous cloné dans pBR322. Ce fragment comporte les derniers nucléotides de la séquence codant pour la p15E de l'enveloppe virale, l'intégralité du Long Terminal Repeat (ou LTR) et les 3/5 èmes des séquences gag. Il constitue la matrice de l'architecture du vecteur.

Après remplacement du site ClaI par un site EcoRI, le fragment EcoRI-PvuII a été sous-cloné respectivement en EcoRI-SmaI de l'adapteur multiple de pUC19. Ce clone a, d'une part, été maintenu intact pour former l'architecture de base du vecteur incluant : le LTR d'amont ou LTR 5', le site de fixation de l'initiateur de la transcription virale (Primer Binding Site ou PBS), la séquence d'encapsidation, les séquences gag et le segment de l'adaptateur multiple (polylinker) de pUC19 ménagé par la digestion EcoRI/SmaI ; destiné à l'insertion de gènes d'intérêt.

D'autre part, un fragment BamHI-BamHI a été dérivé, en remplaçant en amont, le site EcoRI par un site BamHI ; et en tirant profit, en aval, du site BamHI endogène du virus, situé immédiatement en aval du site donneur d'épissage. Ce fragment a été introduit dans l'armature de base, dont le site HindIII du polylinker avait été au préalable remplacé par un site BglII, générant des extrémités cohésives avec celles générées par l'enzyme BamHI.

Le gène marqueur dérivé du Rétrotransposon Tn5, conférant une résistance à la Néomycine (NéoR), a été introduit entre les deux LTRs. Après transformation sur une souche de bactéries hypercompétentes de phénotype recombinase négatif profond, afin de prévenir des remaniements éventuels des séquences en présence, les transformants de la configuration attendue ont été sélectionnés sur la base d'une carte de restriction étendue explorant la totalité de la construction. L'un d'entre eux, baptisé pFOCH29 a ensuite été amplifié et purifié afin de disposer d'une source de matériel adéquat, destiné à la transfection de lignées auxiliaires productrices de particules virales.

### 2- Obtention de clones producteurs de virus défectif

Transfection de lignées d'encapsidation psi-CRIP : Le plasmide pFOCH29-Néo a été introduit dans la lignée d'encasidation amphotrope psi-CRIP décrite par Danos et al. (1988) par transfection utilisant la précipitation au phosphate de calcium selon la procédure standard, sans ADN carrier.

Après la mise sous sélection par la Généticine, 40 parmi les colonies apparues ont été prélevées et le surnageant de culture utilisé pour infecter des fibroblastes murins (NIH3T3) Le processus primaire de sélection d'une série de clones les plus hautement producteurs de particules virales défectives encapsidées a reposé sur l'utilisation de la technique d'amplification génique par réaction en chaîne de la polymérase. L'intégration virale est analysée par PCR sur un lysat de NIH3T3 parvenues à confluence.

Deux couples distincts d'amorces de PCR ont été utilisés : 1° un premier couple amplifiant le segment terminal des séquences gag incluses dans la construction et les deux tiers proximaux du gène de résistance à la néomycine ; 2° un second couple d'amorces amplifiant le tiers distal du gène de résistance à la Néomycine et la moitié du LTR d'aval (3').

Quatre clones ont été retenus sur la base d'un signal de PCR plus intense que les 36 autres ; la répétition de la procédure a permis de confirmer les données initiales indiquant que pour deux clones le signal se détachait de façon nettement plus intense. Ces deux clones ont été amplifiés et le surnageant de culture des cellules productrices a ensuite été utilisé pour infecter des NIH3T3 à plus large échelle dans le but d'évaluer l'efficacité de la construction en termes quantitatifs.

### 3- Evaluation des clones producteurs

### PCR Quantitative

Une analyse par PCR semi-quantitative a été menée en utilisant le couple d'amorces amplifiant la région correspondant au tiers distal du gène NéoR jusqu'à la partie médiane du LTR d'aval (3'LTR). Pour chaque clone 1µg et 3µg d'ADN génomique extrait à partir de cellules NIH3T3 infectées par un surnageant pur après ou sans sélection par la Néomycine, ont été utilisés. Chaque test a été effectué en double (duplicate). Plusieurs dilutions du plasmide pFOCH29-Néo ont été testées en parallèle calculées de telle sorte qu'elles correspondent à 0.1, 0.5 et 1 copie du transgène pour l'équivalent de 1µg d'ADN génomique soit respectivement 0,115 pg, 0,575 pget 1,15 pg pour un plasmide d'une taille de 7164 pb.

La PCR a été réalisée sur 24 cycles, ce qui correspond à une phase encore exponentielle de la réaction. La lecture a été réalisée par analyse densitomètrique informatisée (Cybertech) de la fluorescence au bromure d'éthidium (cf tableau).

Pour le premier clone, une différence significative a été observée entre l'intensité du signal obtenu à partir des cellules infectées sélectionnées et non sélectionnées ; plus franche sur les échantillons de 1µg (70% du signal par rapport au sélectionné) que sur ceux de 3µg pour lesquels le système de détection est saturé par l'intensité du signal.

Pour le deuxième clone, il n'existe aucune différence d'intensité du signal entre cellules sélectionnées et non-sélectionnées, ni pour les échantillons de 1µg, ni pour ceux de 3*µ*g. Ceci suggérant qu'un pourcentage proche de 100% des cellules NIH3T3 ont pu être infectées par le surnageant pur de ce clone producteur. Le haut degré d'infectivité de ce clone était par ailleurs suspecté par l'observation d'une absence de mortalité cellulaire lors de la mise sous sélection par la Néomycine des NIH3T3 infectées avec le surnageant de culture.

### Southern Blot

L'ADN des cellules NIH3T3 infectées a été soumis à une hydrolyse avec deux enzymes de restriction : KpnI et Pst I. Selon les sondes utilisées, la taille des bandes attendue après intégration virale varie. Pour l'enzyme KpnI qui coupe à l'intérieur des LTRs et au milieu de l'adaptateur multiple de pUC19, une sonde LTR révèle un fragment de taille constante de 3610 pb et deux fragments de taille variable selon la proximité de sites KpnI génomiques endogènes à proximité du site d'intégration ; une sonde dérivée par PCR avec les amorces tiers distal NéoR/segment proximal LTR, un fragment de même taille (3610 pb) est attendu, et un seul des deux autres fragments variables d'une intégration à l'autre.

Pour l'enzyme PstI qui coupe deux fois dans la partie médiane de gag et une fois dans l'adaptateur multiple de pUC19, après intégration les fragments allumés par les deux sondes précédentes seront de taille variable ; une sonde générée par PCR à partir du second couple d'amorces allume un fragment constant de 790 bases.

Plusieurs dilutions du plasmide pFOCH29-Néo digéré par KpnI et PstI ont été adjointes sur le Southern blot ; ces dilutions correspondant respectivement à 0.1, 0.5 et 1.0 copies du plasmide pour 10 *µ*g d'ADN génomique.

En outre, l'ADN de cellules infectées non sélectionnées après l'infection et des mêmes cellules sélectionnées par la Néomycine a été systématiquement juxtaposé afin de quantifier l'infectivité de la construction ; les cellules ayant fait l'objet d'une sélection constituant un témoin d'infection de 100%.

### Titration : Titres infectieux viraux par dilutions virales

Des dilutions successives du surnageant primaire ont été utilisées pour infecter des NIH3T3 ; surnageant pur, dilué au 1/10ème, au 1/1000ème et au 1/100 000ème. Les cellules sont infectées par du surnageant viral, à raison de 0,5 ml sur un puits de 35 mm de diamètre : la drogue de sélection est ajoutée dès 20 heures après l'infection directement sur la boîte de culture, sans trypsiniser les cellules. Le titre infectieux retenu correspond au nombre de colonies observées pour la dernière dilution où des colonies apparaissent que multiplie l'inverse de cette dilution.

Pour le premier clone, le titre des cellules productrices initiales est de 2.10⁶ pfu/ml. Pour le deuxième clone, le titre est de 10⁶ pfu/ml

Les deux clones producteurs ont été d'une part congelés régulièrement afin d'en conserver des passages initiaux ; et d'autre part, maintenus en culture continue sur plusieurs mois. Les titrations successives (dilutions du surnageant viral) ont permis d'identifier une baisse progressive des titres. Pour le premier clone, le titre est passé de plus de 2.10⁶ à seulement 10¹ en l'espace de deux mois de culture continue ; cette chute drastique du titre s'est accompagnée d'une modification de la pousse des cellules en culture avec un aspect en foyer et un décollement facile. Pour le second clone, le titre est passé de plus de 10⁶ à 10⁵ en l'espace de deux mois de culture continue, pour diminuer jusqu'à 10³-10⁴ après trois mois et demi ; cette chute modérée du titre ne s'est accompagnée d'aucune modification de la morphologie ni de la pousse des cellules en culture.

### 4- Test activité helper sur 3T3 BAG

Cette recherche a été effectuée par test de mobilisation sur lignées 3T3BAG (Danos et al, 1988 ; Danos, 1991).

Les cellules 3T3BAG ont dans un premier temps été infectées par le surnageant pur de lignées d'encapsidation infectées. Plusieurs cycles successifs d'infection de 3T3BAG vierges avec le surnageant des 3T3BAG précédemment infectées ont été pratiqués pour sensibiliser le test, qui s'est révélé négatif. En outre, aucune colonie de cellules résistant à la Néomycine n'a pu être détectée après mise en contact de NIH3T3 vierges avec le surnageant de NIH3T3 infectées et sélectionnés par la Néomycine.

### 5) SITES D'INTEGRATION

Des cellules de primate humain et non-humain ont été utilisées pour identifier le nombre de sites d'intégration du virus après infection. Les cellules murines fournissent des indications de qualité très moyenne, dans la mesure où il existe sur ces cellules un bruit de fond significatif lié aux multiples intégrations de séquences rétrovirales ou rétrovirales-like.

A cette fin, des cellules VERO de singe ont été infectées avec plusieurs dilutions de surnageant viral. Pour la dilution 10⁻², des clones indépendants ont été obtenus ; chacun ayant été initié à partir d'un seul profil d'intégration virale. L'utilisation d'une enzyme de restriction coupant d'une part à l'intérieur de la construction virale et d'autre part, dans l'ADN génomique de la cellule hôte à des distances variables des sites d'intégration, a permis d'obtenir autant de fragments de restriction, de taille variable, qu'il y a eu d'événements d'intégration. Ici, les enzymes XbaI ou SalI ont été utilisées.

### 6) STABILITE DES TITRES VIRAUX - MASTER BANK SYSTEM

Un stock homogène de cellules productrices de virus a été constitué et contrôlé extensivement pour l'absence de virus compétent pour la réplication par les méthodes suivantes :
- Test de Mobilisation sur cellules 3T3BAG
- Amplification sur NIH3T3
- Innoculation à des souris nouveau-né en intrapéritonéal,
   afin d'étudier une éventuelle pathogénèse in vivo.

A partir de cette banque de cellules ("Master Cell Bank" MCB), une banque de cellules de travail (Working Cell Bank) a été constituée. Les titres viraux obtenus sont restés stables sur plusieurs mois et étaient de l'ordre de 20 x 10⁶ à 3 x 10⁷ cfu/ml (les dilutions au départ n'ayant pas été poussées de façon systématique que à 10⁻⁶. Des résultats ont aussi été obtenus, avec un test incluant une dilution à 10⁻⁷. Les titres sont remarquablement stables sur plusieurs mois à ce niveau d'intensité.

### DISCUSSION

### 1_{°} Construction du virus

La construction virale a été basée sur le principe de conservation des séquences critiques comme le PBS, la séquence d'encapsidation, le site donneur de splice, et aussi sur la conservation d'un long segment de gag, dont le maintien participe à la stabilisation des transcrits comme l'ont montré plusieurs auteurs (revus par McLachlin et al., 1989).

Plusieurs autres constructions dérivées du même squelette rétroviral ont été réalisées auparavant ; dont une première où le LTR3' était flanqué en amont et en aval de séquences rétrovirales plus longues et où deux adaptateurs multiples de pUC19 et pUC18 étaient en présence. De cette configuration a résulté une grande instabilité, et une infectivité modeste.

Une version similaire à celle de FOCH29 a été construite ; similaire en tous points hormis l'insertion d'une large séquence incluant le site accepteur de splice de la souche "clone 57" du virus de Friend (Sitbon et al). Le plasmide résultant a été baptisé pFOCH29SA-Néo. L'infectivité de cette construction s'est avérée moins démonstrative qu'avec pFOCH29-Néo; Néanmoins cette différence n'a été appréciée que sur les données du screening primaire par PCR.

### 2° Sélection des clones producteurs

La sélection primaire des clones producteurs par la procédure décrite dans la section résultats, exploite la réaction en chaîne de la polymèrase ; les conditions utilisées limitent la capacité de résolution de la méthode à un seuil qui correspond approximativement à un titre rétroviral minimal de 10⁴ pfu/ml. L'inconvénient potentiel de cette procédure est lié au caractère non véritablement quantitatif de la PCR, en particulier pour quarante cycles d'amplification ; en conséquence, il existe une probabilité non négligeable de passer à coté de clones hautement producteurs. Afin de pallier en partie à cet inconvénient, une procédure primaire de sélection basée sur deux couples d'amorces de PCR indépendants a été adoptée.

### 3°- Efficacité de l'infection et Stabilité du virus

L'utilisation de deux couples d'amorces de PCR générant chacun des amplimères de taille attendue, permet en outre de vérifier l'absence de remaniements grossiers du génome viral au sein des segments analysés, après intégration.

Cet élément est au mieux contrôlé par le Southern blot qui vérifie l'absence de remaniements majeurs en l'absence de bandes inadéquates de par leur nombre ou leur taille ; ainsi que l'absence d'évènement d'intégration multiples au sein de cellules dérivées d'un même clone, qui évoquerait la présence de virus helper.

Enfin, l'absence de remaniements majeurs susceptibles d'altérer la construction est au mieux appréciée par le test fonctionnel ; démontrant l'acquisition d'un phénotype résistant à la Néomycine après intégration chromosomique du gène marqueur.

L'efficacité de l'infection a ici été appréciée sur trois paramètres essentiels : 1°) la titration virale conventionnelle qui a permis de démontrer des titres supérieurs ou égaux à 10⁶ pfu/ml.
2°) le Southern blot confrontant l'intensité du signal obtenu après hybridation sur un hydrolysat de 10µg d'ADN génomique provenant de cellules infectées sans sélection et après sélection par la drogue à laquelle le produit du gène transduit confère une résistance. Si des dilutions plasmidiques calculées de manière théorique ont été adjointes, elles constituent un moins bon standard que celui immédiatement sus-mentionné. En effet, les dilutions plasmidiques sont telles qu'une imprécision mineure de manipulation peut conduire à conclure de façon erronnée à une différence significative.

L'ensemble de ces éléments, additionné d'une expérience (3°)) de PCR semi-quantitative converge pour indiquer que le surnageant non dilué du second clone infecte les fibroblastes murins avec une efficacité proche de 100%.

Cependant, une instabilité des titres a été constatée avec une perte drastique rapide pour le premier clone et une décroissance beaucoup plus progressive pour le meilleur. Ce phénomène est de constatation banale dans le maniement des cellules productrices qui semblent, au fur et à mesure des passages successifs, perdre leurs capacités d'encapsidation initiales. La production initiale de larges quantités de cellules et leur congélation attentive permet de pallier à cet inconvénient. Néanmoins le titre doit être systématiquement contrôlé, de façon itérative.

En outre, une lignée productrice amphotrope a été choisie. Cependant la transfection initiale de cellules productrices écotropes aurait pu permettre l'infection des lignées amphotropes, éventuellement répétées de façon réciproque et itérative selon la procédure de "ping-pong" (McLachlin JR) (au moyen du surnageant filtré pour prévenir un éventuel mélange des deux types cellulaires) ; celle-ci pourrait contribuer non seulement à augmenter l'infectivité des cellules productrices résultantes, mais aussi à stabiliser les titres rétroviraux. Cependant il a été constaté que systématiquement, des virus helper sont produits et cette méthode ne peut être utilisée chez l'animal ou en clinique.

L'efficacité toute particulière de cette construction mérite d'être soulignée ; d'autant plus que le squelette viral de la souche FB29 du virus de Friend diffère de celui qui a été utilisé pour établir les lignées d'encapsidation. La sécurité des manipulations s'en trouve encore améliorée car les risques de recombinaisons génératrices potentielles de virus compétent pour la replication s'en trouvent encore amoindris.

L'introduction d'un gène marqueur ou de toute séquence d'ADNc sens ou antisens ou d'un fragment d'ADN génomique d'une complexité limitée (nombre d'introns limité) de taille inférieure ou égale à 7kb dont il résultera un ADNc dont les introns seront expulsés dans les cibles, peut être réalisée à partir de la technique décrite précédemment.

Dans le cas où le maintien de séquences introniques semble indispensable à l'obtention de transcrits stables, le fragment génomique d'une taille inférieure ou égale à 7 kb devra être introduit en orientation transcriptionnelle inverse par rapport à la transcription virale, au mieux en utilisant une version de la construction où le LTR(3') sera l'objet d'une délétion de la région U3 et où le fragment génomique sera mis sous la dépendance transcriptionnelle d'un promoteur et/ou d'un activateur de même orientation, afin de ne pas avoir de compétition entre les transcrits sens dépendant des LTR viraux et antisens dépendant du promoteur ajouté.

L'introduction d'une délétion de tout ou partie des séquences promotrices ou activatrices de la région U3 du LTR (3') offre des garanties de sécurité majeure après intégration du rétrovirus. Dans ce cas, l'expression du transgène devra être mise sous la dépendance d'un promoteur et/ou d'un activateur exogène à l'intérieur de la construction.

### 4) - Sites d'intégration

L'étude du nombre de sites d'intégration du virus après infection de cellules de primate a fourni des informations très pertinentes sur la physiologie des virus défectifs à haut titre infectieux et sur la sécurité d'utilisation de ces virus dans des applications thérapeutiques chez l'homme.

### B) CONSTRUCTIONS RETROVIRALES ADDITIONNELLES

### B1) DE CONCEPTION GENERALE ET D'UTILISATION VERSATILE

### 1) - VECTEUR RETROVIRAL S'AUTOINACTIVANT (SIN) ; dénomination : FOCH29-delU3 représenté à la Figure 6.

Compte-tenu de la physiologie de la replication du matériel génétique génomique des rétrovirus, les modifications destinées à la délétion des séquences virales activatrices de la transcription ont été introduites au niveau du LTR en 3' ; la réplication virale à conduit au niveau de la cellule cible de l'infection, à un provirus intégré dont les deux LTRs ont été générés à partir des séquences du LTR initialement en 3'.

Cette délétion a donc été opérée sur le plasmide contenant le fragment BamNI-BglII incluant les séquences du LTR 3'. Le fragment BamHI-BgllI résultant raccourci de 339 bases a été excisé et la ligation avec le plasmide contenant le LTR 5' et les séquences adjacentes effectuée selon la même procédure que pour le vecteur de base. En effet, les séquences activatrices du LTR 5' ont été laissées indemnes pour que la transcription virale puisse avoir lieu et former des transcrits "readthrough" ; des particules virales infectieuses ont ainsi été formées au niveau de la lignée d'encapsidation.

La conception de construction choisie laisse en place la TATA-box mais enlève la CAAT-box (Yu et al, 1986), en utilisant une coupure par l'enzyme BssHII (site unique en 8203 de la séquence du virus natif), qui excise la CAAT-box ; les extrémités cohésives 5' - protubérantes générées par l'enzyme BssHII sont rendues "à bout franc" par remplissage en utilisant le fragment Klenow de l'ADN polymérase I en présence de déoxynucléotides.

L'enzyme de restriction utilisée en amont était :
- soit EspI (= iso-CelII) à la position 7864, située immédiatement après le début des répétitions inversées (IR) (inverted repeats). Les extrémités cohésives 5' -protubérantes générées par l'enzyme EspI ont été rendues "à bout franc" par remplissage utilisant le fragment Klenow de l'ADN polymérase I en présence de déoxynucléotides.
- soit EcoRV, à la position 7984 au milieu des répétitions directes ("direct repeats") que la coupure vient interrompre ; les extrémités générées par l'enzyme EcoRV sont directement à bout franc.

Les extrémités d'amont et d'aval ayant été rendues à bout franc de part et d'autre, une ligation directe a été possible pour refermer la construction, désormais porteuse d'une large délétion de la région U3.

La première version delU3 (délétion EspI en-5'/Iso CelII en 3') a été étudiée en termes fonctionnels : infection et intégration virales et expression résiduelle. La délétion ici créée a emporté la quasi-totalité des séquences U3 du virus. Ceci est idéal en terme de sécurité de la construction rétrovirale.

Nous avons pu montrer que la transcription virale n'était pas altérée dans les lignées d'encapsidation, en introduisant le gène marqueur β-Galactosidase avec un signal de localisation nucléaire dans cette construction. En effet, les cellules productrices ont pris une coloration bleue après transfection ce qui témoigne non seulement de la présence du gène véhiculé par la construction rétrovirale, mais aussi d'une expression de ce transgène.

L'intégration virale a été recherchée par des méthodes moléculaires, en particulier par PCR utilisant les amorces suivantes : Oligo-SENS situé dans le gène B-galactosidase avec la séquence suivante : 5'- CGA CTC CTG GAG CCC GTC AGT ATC -3'; Oligo-ANTISENS situé dans le LTR viral, à cheval entre R et le début de U5 (LTR-508) : 5'- CAG CGA GAC CAC GAG TCG GAT GC -3', dans une région qui est ménagée par délétion EspI/BssHII.

Des constructions alternatives consistent par exemple à inactiver les enhancers viraux, comme pour la délétion avec EcoRV, voire à effectuer des délétions plus courtes conduisant à conserver au virus une activité transcriptionnelle de fond dans les cellules cibles de l'infection. A l'inverse, une délétion emportant outre les séquences décrites dans la version appelée première version de 1U3, la TATA-Box représente un verrou de sécurité supplémentaire ; cependant, des chutes de titres considérables peuvent se produire lors de l'établissement de telles constructions (Yee et al., 1987).

### CONSTRUCTIONS SIN AVEC PROMOTEURS INTERNES

Un promoteur interne utilisable pour réaliser ces constructions est par exemple le promoteur du Récepteur à l'EGF (Epidermal Growth Factor) dérivé du plasmide pERCAT2DE(c) (Maekawa et al 1989) : seules les séquences promotrices d'amont situées entre les nucléotides -2200 à -15 (fragment EcoRI-SstI de 2,2kb) ont été retenues. Les séquences activatrices d'aval contenues dans le plasmide pERCAT2DE(C) n'ont pas été incluses. Un autre promoteur est le promoteur ubiquitaire PGK (Phosphoglycerate Kinase).

### 2) - DECLINAISONS DES CONSTRUCTIONS EXISTANTES

2°-1 Une construction a été faite avec un LTR encore plus épuré en amont des LTR 3' en particulier ; Dénomination FOCH29-PL (pour FOCH29 Pure LTRs) représenté à la Figure 7.

Cette construction a permis d'évaluer le bénéfice en terme de stabilité génétique de l'excision des 140 bases, dont 104 de la fin de l'enveloppe, en amont des LTRs viraux.

La construction a été effectuée à partir du plasmide pUC19 incluant le fragment EcoRI-PvuII décrit dans la partie B1-1 : coupure enzymatique par l'enzyme de restriction EspI (ou IsoCelII) en position 7864 (soit +23 du LTR viral). A l'extrémité 5' les bases générées par une coupure EcoRI ont été artificiellement ajoutées sur un oligonucléotide de synthèse double brin complémentaire des 23 bases du LTR (140 bases dont 103 bases d'enveloppe) . En 3' l'oligonucléotide est complémentaire des extrémités cohésives SpeI. Les séquences des oligonucléotides sont les suivantes : Oligo-SENS 5' - AAT TCA ATG AAA GAC CCC AAA TTG C -3' ; Oligo-ANTISENS 5' - TAA GCA ATT CGG TGG GGT CTT TCA TTG -3'.

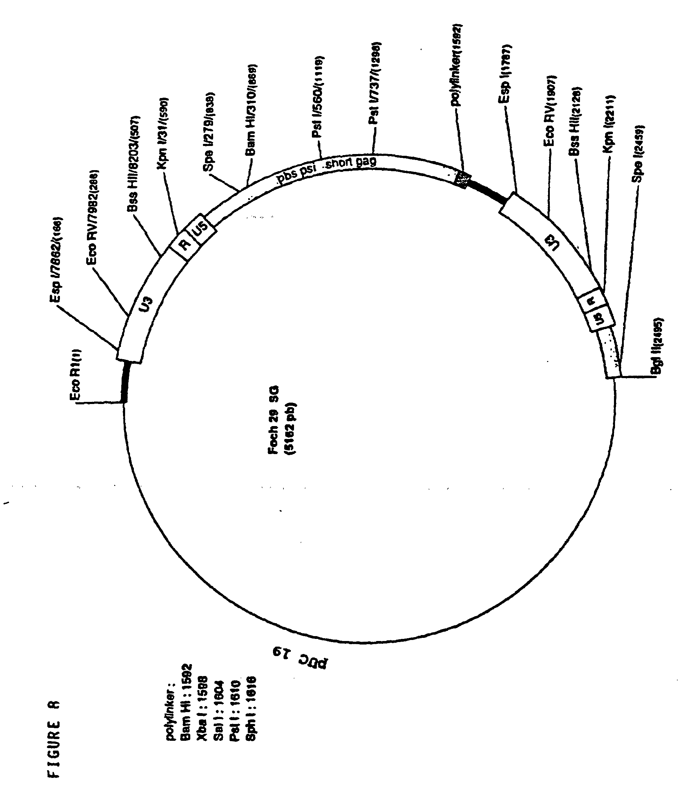

2°-2 GAG raccourci, en maintenant intactes les séquences d'encapsidation ; dénomination FOCH29-SG (pour FOCH29 Short GAG) représenté à la Figure 8.

La construction FOCH29 de base laisse en place la moitié des séquences GAG ; la transcription et vraisemblablement la traduction de MAp15 et de pp12 étant maintenues.

Le maintien d'une portion significative de GAG a été décrit comme pouvant bénéficier à la stabilité de la construction rétrovirale. Ceci peut représenter cependant un inconvénient en terme de place disponible pour les séquences exogènes à vectoriser, d'une part ; et d'autre part, en terme de sécurité de la construction, la probabilité de favoriser des événements de recombinaison avec des séquences rétrovirales pour générer des particules virales compétentes pour la replication étant significativement accure.

FOCH29 contient à peine plus de la moitié des séquences GAG de la souche FB29 et présente l'avantage d'une efficacité très remarquable.

Une culture des clones producteurs de FOCH29, réalisée pendant 18 mois n'a pas entraîné la production d'une quantité préjudiciable à la sécurité de l'utilisation, de recombinaisons avec des séquences rétrovirales générant des particules virales compétentes pour la réplication.

Dans cette perspective d'améliorer la sécurité virale, une construction alternative a été préparée où le GAG de FB29 est coupé au site unique AhaIII (ou isoDraI), à la position 1031 ; un quart seulement des séquences GAG sont alors conservées. La coupure générée par AhaIII étant à bout franc comme pour PvuII utilisée pour le clonage de FOCH29, la construction a été effectuée de façon exactement superposable, le clonage d'amont n'étant pas modifié ; en aval, le site SmaI bout franc du polylinker de pUC19 a été utilisé.

2°-3 Constructions incluant des IRES (pour Intra Ribosome Entry site or Ribosomes Landing Pads) conduisant à des ARN messagers polycistroniques.

Une construction rétrovirale incluant des IRES est en principe destinée au transfert de plusieurs gènes d'intérêt, dont on souhaite que le niveau de transcription soit équilibré puisqu'initialisé à partir d'un seul promoteur (Morgan et al, 1992) ; l'exemple le plus démonstratif est celui du transfert des séquences codant pour les différentes chaînes d'une molécule fonctionnelle dans un contexte hétérodimérique ou trimérique.

Un vecteur polycistronique a été construit pour le transfert des deux chaînes de l'Interleukine 12 ou IL12, p35 d'une part et p40 d'autre part.

Le fragment de la p35 introduit était le fragment suivant: en 5' : PstI à la position +187 de la séquence ; parmi les deux codons ATG (Met) en phase (positions 100-102 et 202-204), celui utilisé était en position 202-204 ; et en 3' : EcoRI à la position +1065 (stop codon TGA en position 904-906).

Le fragment de la p40 introduit était dépourvu de séquences 5' ou 3' flanquantes ; en 5' : site XbaI en position +1 de la séquence (ATG en position +9) ; et en 3' : EcoRI à la position +1007 (stop codon TAG en position 993-995). L'ADN complémentaire de la p40 a un codon AUG optimisé (Séquence Kozak CCATGG ; correspondant au site de restriction NcoI).

Deux types d'IRES ont été utilisés (Borman et al, 1992 ; 1993) ;
- IRES dérivé de poliovirus, qui a nécessité un positionnement précis ; site de fixation du ribosome en 560, AUG fixe à la position 743.
   C'est le fragment KpnI (position +70) / BalI (position +630) qui a été utilisé : soit en amont de p35 (FOCH29-NIRIL12) ; soit en amont du gène de résistance à la Néomycine (FOCH29-IL12-30IR40N).
- IRES dérivé d'EMCV (pour EncephaloMyocarditis Virus), où les séquences IRES dérivées de EMCV nécessitent un positionnement extrêmement précis, en phase, du gène à exprimer. (des séquences IRES dérivées d'EMCV sont commercialisées sous le nom de pCITE pour CAP INDEPENDANT TRANSLATION ENHANCER (Novogen, USA), très voisines de celles qui ont été utilisées ici). Compte-tenu du caractère optimisé de l'AUG de p40, c'est cette chaîne qui a été associée à l'IRES-EMCV quelle que soit la construction ; le fragment d'EMCV utilisé comporte en amont un site EcoRI (position +280) et en aval un site NcoI, situé juste après l'AUG (laisse un résidu "C" qui correspond à la seconde base du quatrième codon) .

Trois constructions ont été réalisées :

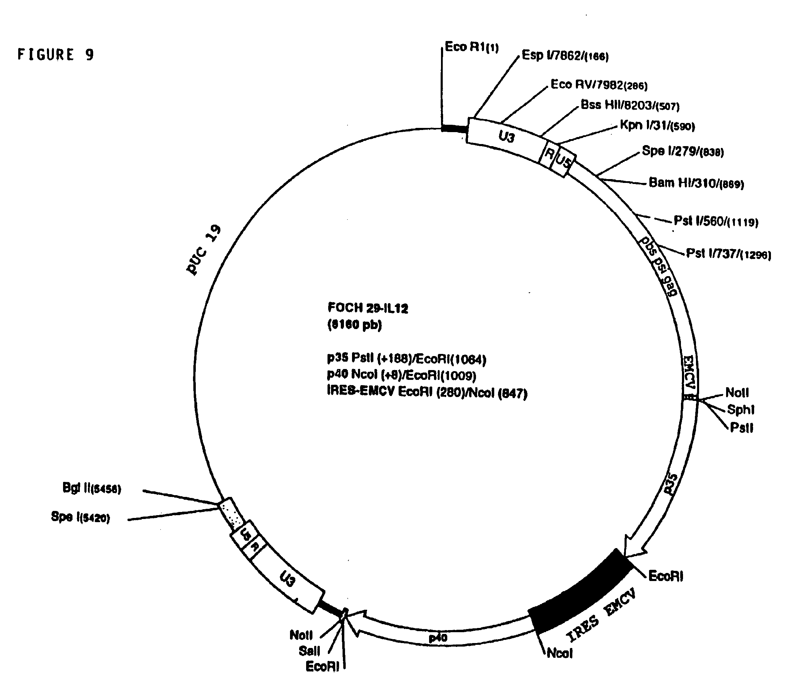

Figure 9 1) FOCH-IL12 : p35 / IRES de poliovirus /p40 : Promoteur LTR.

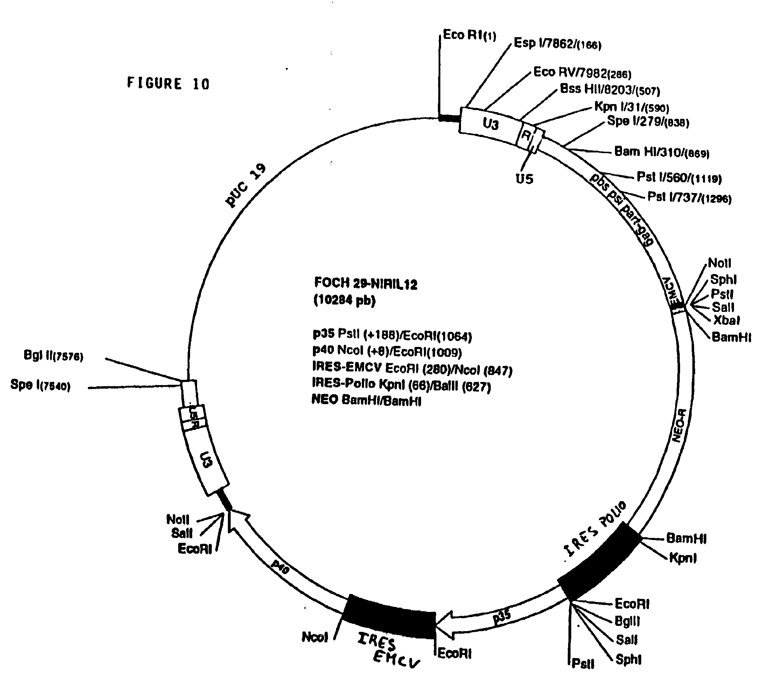

Figure 10 2) FOCH29-NIRIL12 : gène de résistance à la Néomycine / IRES de poliovirus / p35 /IRES d'EMCV / p40.

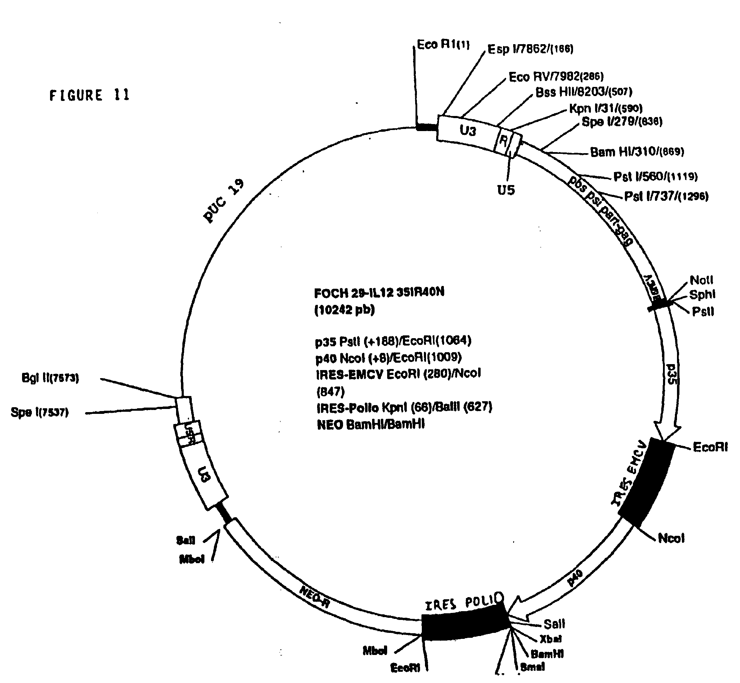

Figure 11 3) FOCH29-30IR40N : p30 / IRES d'EMCV / p40 / IRES de poliovirus / gène de résistance à la Néomycine.

Ces constructions ont toutes fait appel à une construction intermédiaire dans pUC18 dans laquelle :
1°) un site NotI a été introduit à chacune des extrémités du polylinker, en HindIII en amont et en EcoRI en aval ;
2°) un site SalI a été introduit en aval du polylinker entre le site EcoRI et le site NotI
3°) un motif de base ("pUC18-base") a été cloné comportant : p35 / IRES de poliovirus / p40. A partir de ce motif de base, la construction FOCH-IL12 a été obtenue par excision au moyen d'une digestion NotI ; une ligation est pratiquée entre le fragment obtenu et le vecteur FOCH29 ouvert par NotI, pour lequel un linker a été introduit au site XbaI.

Les constructions FOCH29-NIRIL12 et FOCH29-IL12-30IR40N, ont été obtenues après adjonction respectivement : du bloc gène de résistance à la Néomycine / IRES de poliovirus au site SphI en amont du "pUC18-base" ; et du bloc IRES de poliovirus / gène de résistance à la Néomycine au site SalI en aval du "pUC18-base".

2°-4 Construction GAG-POL pour contribuer à la mise au point d'une lignée d'encapsidation originale.

Les protéines de nucléocapsides et la transcriptase inverse sont dérivées des séquences de la souche FB29 dans la lignée de complémentation.

Les lignées d'encapsidation actuellement disponibles et dérivées de cellules murines, présentent les inconvénients suivants :
- Co-encapsidation, concomitante des constructions défectives, de séquences rétrovirales endogènes (MCF, VL30, voire rétro-transposons). Ces séquences co-encapsidées sont donc susceptibles elles aussi de s'intégrer, après infection des cellules cibles du transfert.
- Expression de protéines de complémentation, en particulier l'enveloppe, pilotée dans ces lignées par un LTR rétroviral. Bien que les lignées de troisième génération utilisent des constructions rétrovirales de complémentation, comprenant plusieurs sites de mutation ou délétion, le maintien de séquences LTR est en soi un inconvénient potentiel ; en effet, elles sont susceptibles de donner lieu à une recombinaison génétique avec les constructions défectives à complémenter.

L'ensemble de ces éléments ont conduit à tenter d'améliorer la sécurité des conditions de transfert génétique avec des vecteurs rétroviraux. La lignée d'encapsidation est développée sur le principe des lignées de troisième génération ; c'est-à-dire, avec fragmentation des séquences codant pour les protéines de complémentation, en deux parties (deux étapes de transfection successives).

2°-4-1 Dans une première étape, les étapes impliquant l'utilisation des séquences dérivées de la souche FB29 du virus de Friend sont décrites : en particulier, développement de la cellule de base "DOGP29".

DOGP29 a été obtenue par transfection de la construction LTR-SD-Délétion Psi-GAG / POL détaillée ci-après (et co-transfection avec gène de sélection, résistance à la phléomycine) sur cellule foetale de chien optimisée selon les critères suivants : 1-Absence de rétrovirus endogènes ; 2- Cellule adhérente ; 3- Croissance rapide ; 4- Morphologie stable et homogène ; 5-aisément transfectable ; 6- Nombre de passages supporté très élevé (passages artificiels intensifs pour test) ; 7- Eventuellement capable de soutenir LTC-IC (Hémato).

Un master Cell Bank System est réalisé à partir du clone de cellules de chien retenu selon l'intensité de synthèse des protéines virales de complémentation et la stabilité d'expression de la reverse transcriptase (POL).

La construction gag/pol complémentant pour les protéines de nucléocapside et la transcriptase inverse est dérivée de la souche FB 29 du virus de Friend.

La construction de base a été réalisée à partir de la construction décrite au paragraphe où le LTR était laissé en place ou remplacé par les séquences du promoteur RAR-β.

Une délétion large des séquences d'encapsidation situées en amont des séquences codant pour les nucléoprotéines de capside gag (commençant en + 619) est pratiquée comme suit : coupure SpeI (ou IsocelII), site unique en + 280 ; et PstI en + 560-561 qui enlève 280 bases. Un adaptateur synthétique SpeI-PstI est synthétisé : 5' -CTAGTGCA-3' et apparié ("annealing") au plasmide, recoupé par HindIII. Une ligation est ensuite pratiquée avec le troisième fragment PstI-HindIII incluant la majeure partie des séquences GAG et POL.

Puis, dans un deuxième temps (après transformation et sélection des recombinants positifs), le fragment PstI-PstI (561-737), incluant l'ATG du GAG a été cloné au site PstI dans sa position originale, afin de rétablir l'intégralité des séquences GAG ; l'orientation du clonage de ce petit fragment symétrique PstI-PstI est établie par une digestion enzymatique HaeII (position 720 ; un second site est situé en 4291, mais ne gène pas l'orientation) / AhaIII (site unique sur toute la séquence FB29, position 1031) on EspI (site unique sur toute la séquence FB29, position 7864 du LTR). Du fait de la méthode utilisée pour le clonage au site HindIII de pUC19 des séquences d'ADN correspondant à la totalité du génome de FB29, la fin des séquences POL a été récupérée comme suit : coupure initiale par HindIII (5060) et SnaI (= iso-Bst1107I) (site unique en 6335) ; après purification, ce fragment a été recoupé par SmaI (6079), pour obtenir un fragment de 1019 bases, comportant un minimum de séquences d'enveloppe (244 bases). Ce fragment a été sous cloné en HindIII- HincII du polylinker de pUC18. Le signal de polyadénylation de SV40 a été juxtaposé en aval (excisé du plasmide pCRIPgag-2, Danos et Mulligan, 1988) de la construction. Les séquences POL et Poly-A ont été excisées en bloc de pUC18 et une ligation a été pratiquée avec le plasmide pUC19/LTR épuré/del-Psi/GAG/2/3-POL, décrit au paragraphe ci-dessus.

2°-4-2 Les séquences d'enveloppe amphotropes conventionnelles, similaires à celles utilisées dans la lignée psi-CRIP ont été utilisées pour la complémentation en enveloppe, avec une transcription sous 5'LTR viral avec site de polyadénylation d'aval de SV40, comme dans la lignée Psi-CRIP (Danos et Mulligan, 1988).

### B.2 INCLUANT DES GENES D'INTERET

Qu'il s'agisse de constructions :
- utilisant le vecteur de base avec ses LTRs natifs ou la version SIN délétée des enhancers viraux
- utilisant divers promoteurs internes pour les constructions dérivées du vecteur SIN
- positionnant le gène d'intérêt en orientation transcriptionnelle sens ou antisens par rapport à la transcription virale

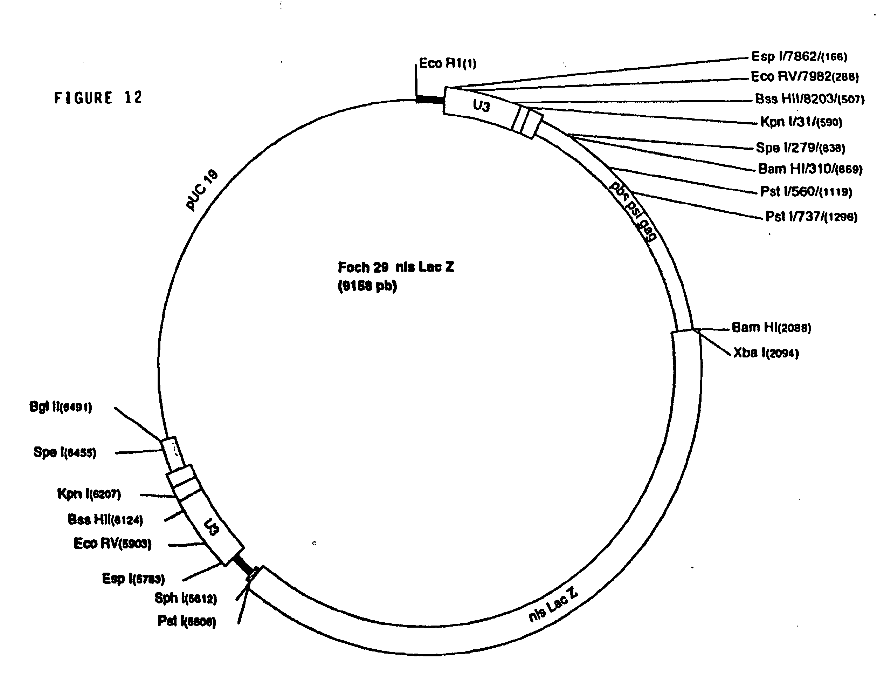

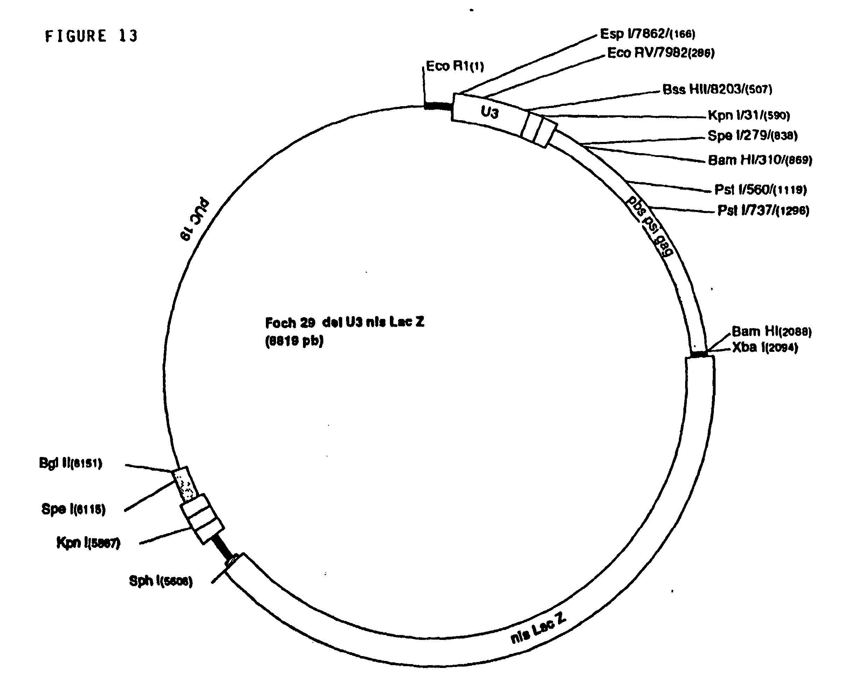

1°- ADNc codant pour la B-Galactosidase avec un signal de localisation nucléaire (Figures 12, 13)

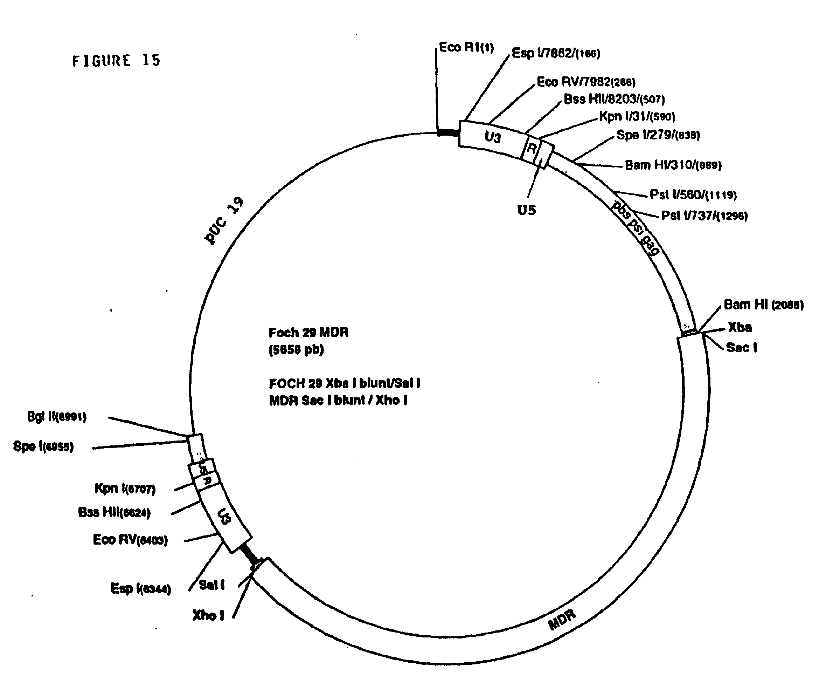

2°- ADNc codant pour la gp170, dérivé du gène de résistance pleiotropique aux drogues cytotoxiques (ex insert dérivé de pMDR1) (Figure 15)

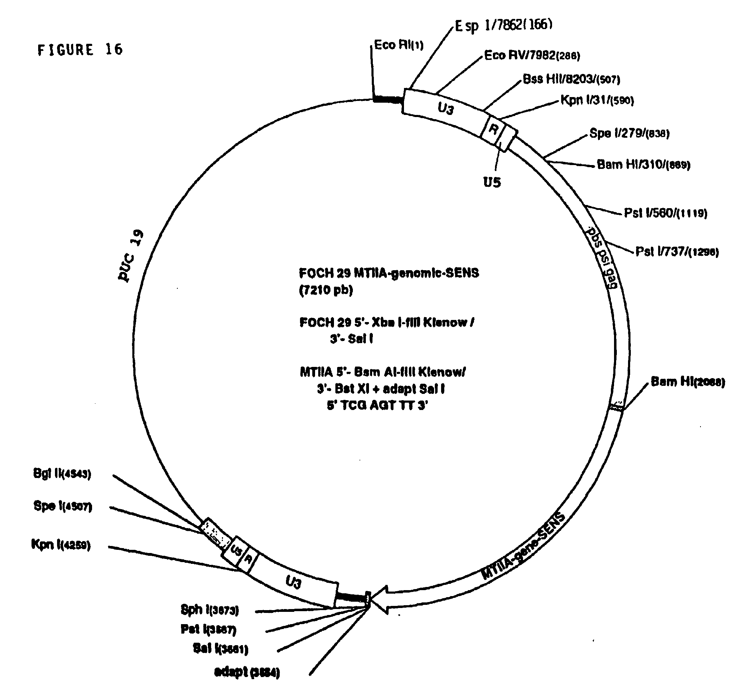

3°- Séquences génomiques et ADNc codant pour la Métallothionéine IIA (Figures 16, 17, 18, 19)

4°- ADNc du gène FACC, avec ou sans ses éléments promoteurs, déficient chez les patients atteints de Maladi de Fanconi du groupe de complémentation C

Le squelette rétroviral FOCH29 a été utilisé :
a)- soit dans sa version native, avec une transcription définie par l'activité des Long Terminal Repeats (LTRs) viraux. Cette construction permet d'évaluer l'efficacité des LTRs du virus de Friend à exprimer le gène FACC dans les cellules hématopoiétiques.
b)- soit dans la version del-U3 de l'enhancer viral qui permet de tester le bénéfice d'une commande transcriptionnelle par un promoteur interne dérivé d'un gène de fonctionnement ubiquitaire, avec un fonctionnement de base modeste mais stable : il s'agit soit du promoteur du gène FACC lui-même, soit du promoteur MT IIA (MétallothioneineIIA), soit du promoteur PGK (Phospho-Glycerate Kinase). La construction utilisant le promoteur du gène FACC est préférée à condition que le niveau d'expression obtenu puisse être compatible avec une correction phénotypique.

### Choix de l'ADNc :

Trois ADN complémentaires différents correspondant à trois types d'ARN messagers ont été clonés pour le groupe de complémentation C de la Maladie de Fanconi ; le cadre ouvert de lecture est identique quelque soit l'ADN complémentaire considéré (Strathdee et al, 1992).

L'un des messagers est très largement majoritaire, sur les cellules en culture. Son extrémité 5' ne comporte qu'une partie de l'exon -1 ; par contre, l'extrémité 3' non-condante est très étendue. Les séquences 3' flanquantes semblent avoir une importance décisive pour la stabilité des transcripts.

Quel que soit l'ADN complémentaire choisi, celui-ci est excisé par coupure BamHI/XhoI et introduit dans les constructions rétrovirales, en orientation antisens comme suit : en amont, XhoI est cohésif des extrémités générées par coupure SalI ; en aval, le site SphI est rendu à bout franc adapté au site BamHI lui-aussi lissé.
1- L'ADN complémentaire majoritaire de 4,5 kb (taille qui représente grossièrement la limite supérieure pour un vecteur rétroviral).
2- L'ADN complémentaire à l'un des deux autres messagers a été en outre utilisé, en orientation sens à l'intérieur de la construction rétrovirale enhancer-free (del-U3), afin de tester le bénéfice potentiel de l'utilisation des séquences 5' flanquantes et de l'exon -1 pour l'expression du gène FACC ; le clonage est alors réalisé par coupure XbaI rendu blunt en amont pour s'adapter à BamHI-blunt, l'adaptation SalI-XhoI se faisant en aval.
3- Enfin, plus simplement, l'ADN complémentaire correspondant aux régions codantes du gène FACC, dépourvu des régions flanquantes en 5' et en 3', a été introduit :
   - soit dans le vecteur rétroviral natif, en orientation sens avec une transcription gouvernée par les LTRs rétroviraux
   - soit dans le vecteur rétroviral enhancer-free, en orientation sens, avec une transcription gouvernée par un promoteur ubiquitaire Métallothioneine ou PGK (Phosphoglycerate-kinease).

5°- ADNc du gène PLP, déficient chez les patients atteints de Maladie de Pelizaeus-Metzbacher (Dautigny et al., 1986 ; Hutson LD et al., 1989 ; Morello et al., 1986).

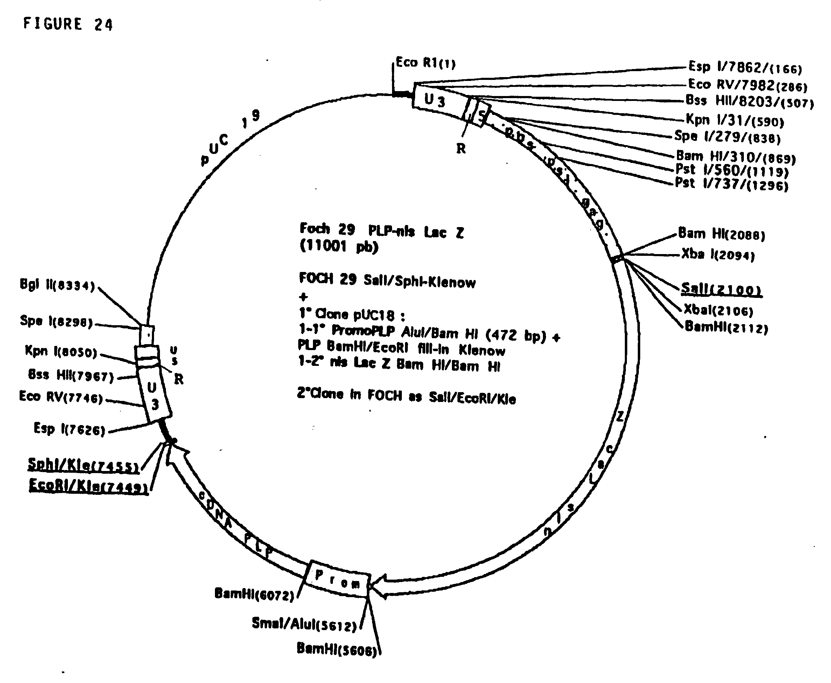

Une construction (Figure 24) a été réalisée pour l'instant, afin à la fois d'exprimer le gène PLP sous son promoteur naturel hautement spécique tropique et inductible par les glucocorticoides ; maisaussi de pouvoir suivre des oligodendrocytes ou des cellules de Schwann exprimant PLP in vivo, après implantation stéréotaxique dans le cerveau.

A cette fin le gène codant pour 1a B-galactosidase muni du signal de localisation nucléaire (nls-LacZ) a été placé sous dépendance immédiate du LTR. Les séquences Promoteur-PLP/ADN complémentaire-PLP sont placées en aval dans le polylinker. Le fragment BamHI-BamHI de nls-LacZ a été cloné dans le site BamHI du polylinker de pSPT18. Les fragments AluI / BamHI et BamHI / EcoRI (traité à la Klenow en présence de dNTPs) incluant respectivement le promoteur PLP et l'ADN complémentaire PLP ont été clonés après ligation aux sites SmaI en amont (pour AluI) et EcoRI en aval, dans pSPT18.

L'ensemble de l'insert est excisé par EcoRI en aval, dont l'extrémité est traitée à la Klenow (bout franc) puis SalI, pour l'amont ; et adapté au polylinker de FOCH29, en amoint en SalI et en aval en SphI traité à la Klenow (bout franc).

D'autres constructions dans la version del-U3 de FOCH29 sont prévues, incluant en orientation inverse le promoteur de PLP suivi d'une séquence comprenant le premier intron du gène associé à l'ADN complémentaire. La présence de cet intron devrait améliorer l'expression et la stabilité des transcrits et permettre un épissage alternatif éventuel PLP-DM20.

6°- ADNc de chacune des chaînes de l'Interleukine 12 : p35 et p40, dans un vecteur polycistronique, construction décrite plus haut, comme exemple de construction comportant des IRES.

D'autres constructions peuvent être obtenues en incluant par exemple les gènes suivants :
7°- TIMP : Tissue Inhibitor of Metalloproteinases (inhibiteur tissulaire des métallo-proteinases)
8°- TNF : Tumor Necrosis Factor (facteur de nécrose tumorale)
9°- IFN-gamma : Interféron-gamma
10°- IFN-B : Interféron-bêta
11°- gènes de cytokines, comme par exemple, l'interleukine

### C- CIBLES CELLULAIRES

Les vecteurs de l'invention ont été utilisés pour transfecter différentes cellules souches. A titre d'exemple on cite les cellules suivantes :

### a- CELLULES SOUCHES HEMATOPOIETIQUES D'ORIGINE HUMAINE

Le vecteur rétroviral FOCH29 a initialement été construit dans le but de rechercher une souche virale susceptible d'infecter plus efficacement les cellules souches hématopoiétiques et de conduire à une expression significative, plus élevée des gènes d'intérêt dans ces cellules qu'avec les vecteurs couramment utilisés. Cet effet présumé étant attendu du fait d'un tropisme particulier des séquences régulatrices virales, en particulier séquences U3 des LTRs.

Le gène de résistance à la Néomycine (Néomycine phosphotransfèrase dérivé du transposon Tn5) a été introduit pour former le vecteur FOCH29-NEO décrit précédemment et utilisé comme gène marqueur.

Pendant une année, on a étudié les conditions optimales de transduction de progéniteurs hématopoiétiques d'origine humaine CD34+, sélectionnés selon diverses méthodes, dérivés soit : 11) de sang de cordon ombilical, 21) de moëlle osseuse (greffe allogènique), 31) de cellules souches du sang périphérique mobilisées par une combinatoire de chimiothérapie et de facteurs de croissance.

Deux ordres de problématiques ont été envisagés :
1- Comparaison de différentes procédures d'infection virale
   1-1. Utilisation de surnageant viral versus coculture (sur des cellules d'encapsidation produisant le virus en continu)
   1-2. Stimulation des cellules par différentes combinatoires de facteurs de croissance
2- Evaluation du succès du transfert :
   2-1. dans l'absolu, quel que soit le stade de différenciation des cellules transduites, par des méthodes biologiques et moléculaires
   2-2. dans des cellules susceptibles de représenter des cellules pluripotentes, avec la mise au point de cultures à long terme, en particulier sur stroma xénogénique d'origine murine, et analyse séquentielle de la clonogénicité des cellules

Les résultats (détaillés plus-bas) obtenus, en particulier à partir de surnageants viraux, confirment l'hypothèse initiale d'une efficacité singulière du vecteur rétroviral sur les précurseurs hématopoiétiques.

Le détail des protocoles expérimentaux, ainsi que les résultats obtenus sont décrits :

### PROTOCOLES EXPERIMENTAUX

### 1- COMPARAISON DE DIFFERENTES PROCEDURES D'INFECTION VIRALE

### 1-1.Utilisation de surnageant viral versus coculture sur des cellules d'encapsidation produisant le virus en continu

En effet, l'utilisation de cocultures présente des inconvénients majeurs pour les applications humaines relatifs :
1) à la contamination potentielle des cellules hématopoiétiques par des cellules xénogéniques et
2) à la persistance potentielle d'une multiplication de ces cellules productrices malgré leur irradiation (ou leur traitement par des agents cytotoxiques) préalable à la mise en oeuvre de la coculture.
3) à l'augmentation significative du risque de génération de virus compétent pour la replication, du fait de la présence de séquences complémentant les virus défectifs, susceptibles de fournir des événements de recombinaison homologues et non homologues avec des séquences rétrovirales endogènes ou virus contaminants fortuits des milieux de culture (Temim et al, 1990).

Une comparaison en parallèle de l'infection d'une même source cellulaire unique (progéniteurs CD34+ sélectionnés soit à partir d'une unique poche de sang de cordon ombilical ; soit à partir d'une cytaphérèse) séparée en deux échantillons équivalents en tous points a été faite ; l'un d'entre eux était mis en contact avec une sous-couche adhérente de cellules productrices de virus, menées à 80% de confluence sur de la gélatine à 1%, en présence de polybrène à la concentration de 2mg/ml, pendant 48 heures ; l'autre échantillon était placé dans un flacon de culture et recouvert de surnageant viral (sans diluer avec du milieu de culture additionnel), fraîchement récolté à partir de cultures de cellules productrices à confluence et filtré sur membranes de 0,45 µm (éliminant toutes les cellules susceptibles d'avoir été prélevées avec le surnageant), en présence de polybrène à la même concentration. Le protocole d'infection par surnageant viral reposait sur une répétition à raison de quatre cycles sur 36 heures soit deux cycles espacés de huit heures par jour, deux jours consécutifs. Le surnageant a simplement été ajouté sur le puits de culture, sans que les cellules hématopoiétiques ne soient ni manipulées mécaniquement ni centrifugées.

Le milieu de culture utilisé lors de l'infection virale indifféremment du mode d'infection, correspondait au milieu optimisé pour les cellules souches hématopoiétiques, soit DMEM-modifié Iscove (GIBCO-BRL) supplémenté de 10% de sérum de veau foetal (Boehringer-Mannheim) et de 10% de sérum de cheval (GIBCO- BRL).

Chaque paramètre a été testé sur au moins deux puits en duplicate réalisés de façon concomitante.

Après infection , les cellules ont été plantées pour culture à long terme sur stroma xénogénique.

### 1-2. Stimulation des cellules par différentes combinatoires de facteurs de croissance

Des quantités minimisées de facteurs de croissance ont été utilisées, afin de maintenir au mieux la pluripotentialité des précurseurs hématopoiétiques qui pourraient être infectés, l'objectif étant de s'adresser au mieux aux cellules déjà spontanément en cycle.

On a comparé sur une même source cellulaire et pour les mêmes conditions d'infection :

### 1°- DIFFERENTES COMBINATOIRES DE FACTEURS DE CROISSANCE

(Stem Cell Factor : SCF ; Leukemia Inhibiting Factor : LIF ; Interleukine 3 : IL3 ; Erythropoiétine : Epo ; Granulocyte-Macrophage stimulating factor : GM-CSF), SOIT :
- SCF + LIF
- SCF + LIF + IL3
- SCF + LIF + IL3 + Epo
- SCF + LIF + IL3 + GM-CSF

### 2°- DIFFERENTES CONCENTRATIONS DE CES DIFFERENTS FACTEURS

- SCF : 50 ng/ml ; 25 ng/ml ; 10 ng/ml ; 5 ng/ml
- LIF : 10 U/ml ; 5 U/ml ; 1 U/ml
- IL3 : 10 U/ml ; 1 U/ml 0,1 U/ml
- GM-CSF 10 ng/ml

### 2- EVALUATION DU SUCCES DU TRANSFERT

### 2-1. Toutes cellules confondues immédiatement après infection, permettant d'établir un pourcentage initial de cellules transfectées sur la base de méthodes biologiques et moléculaires :

- CFU-GEMM (Test pour la présence de colonies mixtes Granuleuses- Erythroides-Monocytes-Mégacaryocytaires) selon deux modalités :

- sans sélection
- avec sélection pharmacologique par la Néomycine, utilisée à des doses initialement très élevées soit 1mg/ml.
- PCR (réaction en chaîne de la polymérase) sur colonies individuelles à partir des tests CFU-GEMM en méthycellulose repiquées sur les cultures non-sélectionnées ; après repiquage les colonies individuelles ont été amplifiées jusqu'à environ 10⁵ cellules par puits en stimulant avec un cocktail de facteurs de croissance, contenant : SCF 50 ng/ml ; GM-CSF 50 ng/ml ; IL3 10 U/ml ; et Epo 2U/ml. Les cellules ont alors été lysées et analysées (cf protocole et amorces de PCR indiqués dans la partie).

### 2-2. dans des cellules susceptibles de représenter des cellules pluripotentes

Un test de cultures à long terme a été mis en oeuvre, sur stroma xénogénique d'origine murine (lignée MS5), et analyse séquentielle de la clonogénicité des cellules.

Cultures à long terme sur stroma xénogénique, dont la monocouche adhérente a été préparée au préalable sur un film de gélatine à 1%. Ces cultures ont été menées dans des "slide-flask" de 9cm² de surface (Nunc) pendant 60 jours ; chaque semaine, la moitié du surnageant de surface était enlevée et remplacée par du milieu neuf contenant un cocktail minimum de facteurs de croissance (Stem Cell Factor : SCF 5ng/ml ; Leukemia Inhibiting Factor : LIF 5U/ml ; Interleukine 3 : IL3 0,1U/ml et Erythropoiétine : Epo 0,1U/ml).

Les cultures à long terme ont été réalisées en milieu normal ou en milieu sélectif, avec alors un stroma génétiquement manipulé par transfection d'un plasmide PGK-néo pour être rendu résistant à la néomycine.

L'évaluation des cultures à long terme reposait sur :
- l'observation de la présence d'ilôts de cellules hématopoiétiques en surface sur les cellules stromales (Cobblestone)
- la mise en route à des temps précis de tests de CFU-GEMM séquentiels avec ou sans sélection pharmacologique pour la présence du gène de résistance à la Néomycine. les cellules prélevées étaient numérées et plantées à raison de 1000 cellules par puits d'analyse. Les cellules provenaient soit du surnageant de surface communément prélevé chaque semaine, soit d'un surnageant prélevé de façon à décoller aussi les ilôts hématopoiétiques adhérents au stroma (en particulier à J60, lorsque les cultures à long terme sont arrêtées), comme suit : le surnageant a été prélevé en totalité, la boîte de culture a alors été recouverte de PBS 1X sans calcium pendant une à deux minutes ; ce qui a décollé les cellules semi-adhérentes. Ce nouveau surnageant a lui-aussi été prélevé, ajouté au premier et l'ensemble a été centrifugé à très basse vitesse (1000 tours/minute) sur un coussin de sérum de veau foetal qui protégeait les cellules les plus fragiles. Une partie du culot cellulaire était planté pour CFU-GEMM, les autres cellules étant simplement replantées sur un stroma xénogénique pour culture à long terme.
- la caractérisation moléculaire de l'intégration virale a été analysée par tests de PCR séquentiels, sur des colonies individuelles prélevées à partir des CFU-GEMM (voir plus haut).
- Lorsqu'un nombre suffisant de cellules a pu être obtenu au départ et que les cultures long terme restaient très riches une partie des cellules était prélevée pour analyse FACS.

### RESULTATS

### 1- CULTURES LONG-TERME

Les cultures ont été menées avec succès pendant 60 jours sans changer de stroma pendant cet intervalle. A cette date, les cultures ont été arrêtées, et l'ensemble des cellules restantes plantées en CFU-GEMM. Des colonies en test CFU-GEMM planté à 60 jours à partir de cellules sélectionnées en Néomycine ont été obtenues. Ceci atteste de la transduction efficace de précurseurs hématopoiétiques capables d'entretenir une culture à long terme et du maintien d'une expression génique sur plusieurs mois du gène véhiculé par la construction FOCH29 dans les précurseurs hématopoiétiques.

### 2- COMPARAISON DE DIFFERENTES PROCEDURES D'INFECTION VIRALE

### 2-1. Utilisation de surnageant viral versus coculture

On a constaté l'efficacité singulière de quatre cycles itératifs d'infection par surnageant sur deux jours, sans aucune manipulation des cellules par rapport à coculture sur lignée d'encapsidation sur 48 heures.

Ces données ont été authentifiées par :
1° Le maintien de cultures à long terme en présence d'une sélection par la Néomycine sur stroma résistant à la Néomycine ; à J60, les cultures de cellules infectées par surnageant restaient encore productives, alors que les puits infectés par coculture étaient désertiques.
2° Les tests de CFU-GEMM, avec un nombre de colonies, en particulier colonies mixtes, 4 à 5 fois plus nombreuses après infection par surnageant.
3° L'évaluation moléculaire de l'intégration virale par PCR sur colonies individuelles repiquées et amplifiées à partir des CFU-GEMM. Pour les tests initiaux une transduction jusqu'à 90% a été mise en évidence après infection par surnageant.

### 2-2. Stimulation des cellules par différentes combinatoires de facteurs de croissance

Le cocktail de facteurs de croissance (GFs) minimum à des concentrations minimums retenu était le suivant : SCF 10 ng/ml + LIF 10 U/ml, parmi les multiples différentes combinaisons citées au chapitre protocole expérimental.

En effet, les cultures à long terme significatives après infection en présence d'IL3 à des concentrations variant entre 1U/ml et 10U/ml n'ont pas pu être maintenues, du fait d'une expansion initiale significative, sans maintien d'une culture productive à long terme.

En outre, aucun effet du prétraitement par stroma xénogénique en dehors d'une contamination potentielle par des cellules murines n'a été constaté. Ces résultats témoignent donc ;
1° de la faisabilité d'une infection rétrovirale productive sans menacer le potentiel d'initiation de cultures à long terme, à partir d'un surnageant viral et non d'une coculture
2° de l'efficacité à la fois de la transduction virale des précurseurs hématopoiétiques d'origine humaine dérivés des cellules CD34+ au moyen de la construction FOCH29 ; et de l'expression du gène d'intérêt tout au long d'une culture à long terme, à partir de cette construction dans ces mêmes cellules.

Ces éléments attestent de la faisabilité d'applications thérapeutiques au moyen de ce vecteur rétroviral dans les cellules souches hématopoiétiques humaines ; ce qui devrait être confirmé par les résultats d'un protocole d'expérimentation clinique de marquage de greffe.

### b- CELLULES EPITHELIALES

1°- Type cellules de l'épithélium vésical
   - de rat, de chien, de singe
   - d'origine humaine
2°- Type cellules de l'épithélium mammaire
   - de rat
   - d'origine humaine

### c- CELLULES TUMORALES

1°- Type cellules de tumeur vésicale
   - de rat
   - d'origine humaine
2°- Type cellules de tumeur mammaire
   - d'origine humaine

### d- CELLULES ACCESSOIRES DU SYSTEME NERVEUX

1°- Précurseurs d'oligodendrocytes
2°- Cellules de Schwann

Ces deux types de cellules d'origine murine ont été infectées avec la construction FOCH29-NEO afin d'évaluer le tropisme neurotrope des LTRs rétroviraux ; l'expression du gène d'intérêt étant gouvernée à partir des LTRs rétroviraux.

Les oligodendrocytes étaient issus de cultures primaires ; pour les cellules de Schwann, c'est la lignée MSC80 qui a été utilisée. Après infection, les cellules MSC80 ont été maintenues en culture continue sous sélection par la Néomycine pendant quatre mois. La morphologie cellulaire caractéristique s'est maintenue tout au long de cette période de culture prolongée.

La démonstration préliminaire du succès de l'infection de ces cellules neurotropes et le maintien d'une expression satisfaisante du gène marqueur véhiculé par le vecteur FOCH29 a conduit à poursuivre les travaux dans une orientation thérapeutique : la construction du vecteur FOCH29-PLP a alors été réalisée. En effet, ce gène est défectueux chez les patients atteints de maladie de Pelizaeus-Metzbacher (Saugier-Veber et al. 1994). Le bénéfice thérapeutique du transfert rétroviral d'une version normale du gène dans les progéniteurs gliaux responsables de la synthèse de la myéline du système nerveux central, ou dans les cellules de Schwann génétiquement manipulées est évalué dans les modèles murins de la maladie humaine (souris Jimpy, Jimpy/MSD (Pham-Dinh et al., 1992) et Rumpshaker). Nous suivons actuellement le devenir in vivo de cellules MSC80 génétiquement manipulées, après réimplantation stéréotaxique dans le cerveau.

La construction FOCH29-PLP utilise le promoteur endogène du gène PLP ; celui-ci est en outre évalué sur une version delU3 de FOCH29, où la transcription du gène PLP est gouvernée par son promoteur propre.

### e- FIBROBLASTES

1°- d'origine murine
2°- d'origine canine
3°- de primate non humain
4°- d'origine humaine

### f- CELLULES du STROMA HEMATOPOIETIQUE

1°- d'origine murine, en lignée (lignée MS5, utilisée pour les cultures à long terme de cellules souches hématopoiétiques, avec expression sur plus d'une année.
2°- d'origine canine, sur des cultures primaires de fibroblastes médullaires foetaux établies au laboratoire.
3°- de primate non humain
4°- d'origine humaine

### g- CELLULES ENDOTHELIALES

### h- CELLULES MESENCHYMATEUSES

### i- CELLULES MESOTHELIALES

### j- KERATINOCYTES

### k- HEPATOCYTES

### l- LIGNEES d'origine humaine

1°- de LYMPHOCYTES T : JURKATT
2°- de CELLULES NK : YT2C2
3°- de MONOCYTES-MACROPHAGES : U937
4°- ERYTHRO-MEGACARYOCYTAIRE : K562

Ces quatre lignées cellulaires ont été infectées avec un surnageant viral soit par un cycle unique soit par quatre cycles d'infection (deux cycles espacés de huit heures deux jours consécutifs). Seize heures après l'infection les cellules sont mises en sélection par la Néomycine à diverses concentrations : 0,3 mg/ml ; 0,5 mg/ml et 1mg/ml.

Un test de marquage à la thymidine tritiée a été pratiqué une semaine après l'infection (soit après cinq jours de mise en sélection) ; ce test s'est avéré largement positif pour les lignées de croissance rapide : Jurkatt et K562, quelle que soit la concentration de Néomycine utilisée.

Après sélection en Néomycine prolongée de trois semaines, l'analyse comparée de la viabilité cellulaire (cellules en suspension) entre les puits sélectionnés à différentes concentrations et les puits témoins sans sélection, témoigne d'un pourcentage important de cellules effectivement transduites auxquelles une résistance à la Néomycine a été conférée :
LIGNEE TUMORALE DE CELLULES T JURKATT : 75 à 80%
CELLULES NK YT2C2 : 30 à 50%
MONOCYTES-MACROPHAGES U937 : 40 à 60%
ERYTHRO-MEGACARYOCYTAIRE K562 : 75 à 80%

### MODELE DE TRANSFERT RETROVIRAL IN VIVO

### APPLICATION AU CANCER DE VESSIE

La vessie étant un organe creux accessible par une simple sonde endurèthrale, les modalités d'administration et l'efficacité du transfert de gènes par instillation endovésicale directe in vivo ont été évaluées point par point. Cette évaluation comporte plusieurs lignes de paramètres :
1°- Efficacité du transfert rétroviral de l'urothélium vésical, par innoculation endovésicale in vivo de surnageants viraux véhiculant des constructions "reporters".
2°- Innocuité des manoeuvres mécaniques et de la transduction des cellules épithéliales
3°- Absence de diffusion systémique des particules virales
4°- Infection préférentielle des cellules tumorales par rapport à l'épithélium sain

En effet, les tumeurs superficielles de vessie, développées aux dépens de l'urothélium vésical dans 90% des cas, ont une évolution essentiellement marquée par une tendance à la récidive après exérèse complète par résection endoscopique. Un groupe à haut risque de récidive et de progression peut être défini et inclue les tumeurs de stade pTl, les tumeurs multifocales de stade pTa, les cacinomes in situ ou les dysplasies associées, et les récidives tumorales multiples.

Dans ce groupe, la chimiothérapie ou l'immunothérapie non spécifiques par voie endovésicale, sont utilisées comme traitements prophylactiques des récidives après résection endoscopique. Cependant, les traitements endovésicaux actuellement disponibles se sont avérés incapables d'éradiquer les récidives tumorales multiples, voire dans certains cas la progression des tumeurs. Des protocoles thérapeutiques originaux basés sur le transfert de gènes inhibiteurs de l'invasion tumorale (gène TIMP, construction FOCH29) précitée ou capables de stimuler la réponse immunitaire endogène (constructions FOCH29-IL12, FOCH29-IFN-gamma précitées), ou encore interférant avec la transduction des signaux activateurs de la prolifération dans les cellules tumorales, doivent donc être proposés.

### PERSISTANCE DES PARTICULES VIRALES DEFECTIVES ENCAPSIDEES DANS LES FLUIDES BIOLOGIQUES

Dans ce contexte, l'infectivité des virions après incubation à 37°C en présence d'urines (filtrées sur membranes de 0,45µm ou non-filtrées) volume à volume a été analysée, à des temps séquentiels échelonnés comme suit : 5 min ; 10 min ; 15 min ; 20 min ; 30 min ; 45 min ; 60 min ; 75 min ; 90 min ; 120 min ; 150 min ; 180 min. On a pu démontrer, en utilisant le gène marqueur β-gal avec signal de localisation nucléaire, que les titres viraux ne variaient pas sur les trois premières heures ; la pertinence d'une approche par instillation endovésicale in vivo associée à une restriction hydrique s'en trouve renforcée.

### REFERENCES

- Anderson WF, McGarrity, Moen Rc. Report to the NIH Recombinant DNA Advisory Commitee on murine Replication-Competent Retrovirus (RCR) assays (February 17, 1993). Hum Gene Ther, 4 : 311-321, 1993
- Cornetta K. Morgan RA, Anderson WF. Safety issues related to retroviral-mediated gene transfer in humans Hum. Gene Ther. 2 : 5-14, 1991
- Danos O, Mulligan RC
   Proc Natl Acad Sci USA 85 : 6460-64, 1988
- Danos O
   Practical Molecular Virology 17-27
   In Collins M, the Humana Press, Clifton, New Jersey, 1991
- Dautigny A, Mattei MG, Morello D et al. The structural gène coding for myelin-associated proteolipid protein is mutated in Jimpy mice. Nature 321 : 867-875, 1986
- Donahue Re, Kessler SW, Bodine Det Al. Helper virus induced T cell lymphoma in non-human primates after retroviral-mediated gene transfer. J. Exp. Med., 176, 1125-1135, 1992
- French Anderson W
   Human gene Therapy
   Science 256 : 808-813, 1992
- Gunter XC, Khan AS, Nogushi PD. The safety of retroviral vectors. Hum. Gen. Ther, 4, 643-645, 1993
- Hudson LD, Puckett C, Berndt J, Chan J, Genecic S. Mutation of the proteolipid protein gene PLP in a human X chromosome-linked disorder. Proc. Natl. Acad. Sci. USA, 45 : 435-442, 189
- Kessler DA, Siegel JP, Nogushi PD, Zoon KC, Feiden KL, Woodcock J. Regulation of Somatic-cell therapy and gene therapy by the food and drug receptor proto-oncogene. J. Biol. Chem. 264 : 5488-5494, 1989
- McLachlin JR, Cornetta K, Eglitis MA, Anderson WF Retroviral-mediated gène transfer
   Progress in Nucleic Acid Research and Molecular Biology 38 : 91-135, 1990
- Masuda M, Remington MP, Hoffman PM, Ruscetti S. Molecular characterization of a neuropathogenic and nonerythroleukemogenic variant of Friend murine leukemia virus PVC-211. J. virol, 66 : 2798-2806, 1992
- Mathieu-Mahul D, Heard JM, Fichelson S, Gisselbrecht S, Sola B, LarsenC
   Virology 119 : 59-67, 1982
- Miller AD
   Human gene therapy comes of age
   Nature 357 : 455-460, 1992
- Monsigny M. et al
   médecine/sciences 9:441-9, 1993
- Morello D, Dautigny A, Pham-Dinh D, Jolles P. Myelin proteolipid (PLP and DM20) transcripts are deleted in Jimpy mutant Mice. EMBO J 5 : 3489-3493, 1986
- Morgan RA, Couture L, Elroy-Stein O, Ragdeb J, Moss B, French Anderson W. Retroviral vectors containing putative internal ribosome entry sites : developmentof a polycistronic gene transfer systems and applications to human gene therapy. Nucleic Acids Res, 20 : 1293-1299, 1992
- Mulligan RC
   The basic science of gene therapy
   Science 260 : 926-931, 1993
- Perryman S, Nishio J, Chesebro B
   Complète nucleotide sequence of Friend murine leukemia virus, strain FB29
   Nucl Acid Res 19 : 6950, 1991
- Remington MP, Hoffman PM, Ruscetti S, Masuda M. Complète nucleotide séquence of a neuropathogenic variant of Frien murine leukemia virus PVC-211. Nucl Acids Res, 20 : 3249, 1992
- Saugier-Veber P, Munnich A, Bonneau D, Rozet JM, Le Merrer M, Gil R, Boespflug-Tanguy O. X-linked spastic paraplegia and Pelizaeus-Mersbacher disease are allelic disorders at the proteolipid protein locus. Nat. Genet. 6 : 257-261, 1994
- Sitbon M, Sola B, Evans L, Nishio J, Hayes SF, Nathanson K, Garon CF, Chesebro B
   Cell 47 : 851_859, 1986
- Sitbon M, Ellerbrok H, Pozo F, Nishio J, Hayes SF, Evans LH, Chesebro B
   J Virol 64 : 2135-40, 1990
- Sitbon M, d'Auriol L, Ellerbrok H, André C, Nishio J, Perryman S, Pozo f, Hayes SF, Wehrly K, Tambourin P, Galibert F, Chesebro B
   Proc Natl Acad Sci USA 88 : 5932-5936, 1991
- Strathdee CA, Gavish H, Shannon WR, Buchwald M. Cloning of cDNAs for Fanconi's anaemia by functional complémentation. Nature, 356 : 763-767, 1992
- Temim HM, Safety considérations in somatic gene therapy of human disease with retrovirus vectors. Hum. Gene Ther., 1 : 11-123, 1990
- Yee JK, Moores JC, Jolly DJ, Wolff JA, Respes JG, Friedmann T. Gene expression from transcriptionally disabled retroviral vectors. Proc Natl Acad Sci USA, 84 : 5197-5201, 1987
- Yu SF, von Ruden T, Kantoff PW, Garber C, Seiberg M, Ruther U, French Anderson W, Wagner EF, Gilboa E. self-inactivating retroviral vectors designed for transfer of whole genes into mammalian cells. Proc. Natl Acad Sci USA, 83 : 3194-3198, 1986
- Maekawa T et al, J Biol Chem 264 : 5488-5494, 1989

## Revendications

1. Vecteur recombinant pour le clonage et/ou l'expression et/ou le transfert d'une séquence de nucléotides exogène, **caractérisé en ce qu'**il comprend une séquence comprenant l'enchaînement LTR compris entre les nucléotides 7842 et 144 de la séquence donnée à la figure 1, le site de fixation du primer (PBS) débutant au nucléotide 145, la séquence d'encapsidation comprise dans l'enchaînement de 250 nucléotides suivant la fin de la séquence LTR, ladite séquence étant capable de contrôler l'expression et/ou le transfert de la séquence exogène, quelle que soit son orientation transcriptionnelle par rapport à l'orientation transcriptionnelle du virus.

2. Vecteur retroviral recombinant selon la revendication 1, **caractérisé en ce qu'**il comprend pour l'expression et/ou le transfert d'une séquence de nucléotides exogène toute séquence contenue dans le fragment situé entre les nucléotides 7702 à 310 de la séquence représentée à la figure 1, ladite séquence comprenant les enchaînements LTR5' et LTR3' compris chacun entre les nucléotides 7842 et 144, ainsi que le site PBS débutant au nucléotide 145, la séquence d'encapsidation comprise dans l'enchaînement de 250 nucléotides suivant la fin de la séquence LTR, ladite séquence étant capable de contrôler l'expression et/ou le transfert de la séquence exogène, quelle que soit son orientation transcriptionnelle par rapport à l'orientation transcriptionnelle du virus.

3. Vecteur recombinant selon la revendication 1 ou la revendication 2, **caractérisé en ce qu'**il comprend en outre tout ou partie de la séquence gag située entre les nucléotides 619 et 2245 de la séquence de la figure 5, en particulier la séquence comprise entre les nucléotides 619 et 1527 de la séquence de la figure 1.

4. Vecteur recombinant selon les revendications 1 à 3, **caractérisé en ce qu'**il est essentiellement dépourvu des séquences virales pol et/ou env.

5. Vecteur recombinant selon la revendication 1 ou la revendication 2, **caractérisé en ce qu'**il comprend le fragment comprenant les nucléotides 7702 à 1527 de la séquence représentée à la figure 1.

6. Vecteur recombinant selon l'une quelconque des revendications 2 à 5, **caractérisé en ce qu'**il comprend le fragment comprenant les nucléotides 7702 à 310 de la séquence représentée à la figure 1.

7. Vecteur recombinant selon l'une quelconque des revendications 1 à 6, **caractérisé en ce qu'**il contient en outre au moins un adaptateur multiple présentant des sites de restriction uniques par rapport aux sites contenus dans le vecteur.

8. Vecteur recombinant selon l'une quelconque des revendications 1 à 6, **caractérisé en ce qu'**il s'agit d'un plasmide.

9. Vecteur recombinant selon l'une quelconque des revendications 1 à 8, **caractérisé en ce qu'**il s'agit du plasmide pFOCH29 déposé à la CNCM le 30 juin 1993 sous le n°I-1326.

10. Vecteur recombinant selon l'une quelconque revendications 1 à 9, **caractérisé en ce qu'**il comprend en outre un gène ou une partie d'un gène marqueur, par exemple, le gène de résistance à la néomycine.

11. Vecteur recombinant selon l'une quelconque des revendications 1 à 10, **caractérisé en ce qu'**il comprend une séquence IRES.

12. Vecteur recombinant selon l'une quelconque des revendications 1 à 11, **caractérisé en ce que** la région U3 du LTR est délétée au moins en partie, de façon telle que le promoteur et/ou l'activateur contenu(s) dans U3 est (sont) au moins en partie inactivé(s) ou modifié(s).

13. Vecteur recombinant selon l'une quelconque des revendications 1 à 12, **caractérisé en ce que** la région U5 du LTR est délétée au moins en partie.

14. Vecteur recombinant selon l'une des quelconque revendications 1 à 13, **caractérisé en ce que** la séquence contenue dans le fragment comprenant les nucléotides 7702 à 1527 et/ou ce fragment, et/ou la séquence contenue dans le fragment comprenant les nucléotides 7702 à 310 et/ou ce fragment est remplacée par une séquence hybridant dans des conditions de forte stringence avec la séquence correspondante des susdits fragments.

15. Vecteur recombinant selon l'une quelconque des revendications 1 à 14, **caractérisé en ce qu'**il contient les séquences gag et pol de la souche FB29.

16. Vecteur recombinant selon l'une quelconque des revendications 1 à 15, **caractérisé en ce que** la séquence de nucléotides exogène est sous le contrôle d'un promoteur exogène.

17. Vecteur recombinant selon l'une quelconque des revendications 1 à 16, **caractérisé en ce qu'**il est introduit dans une lignée d'encapsidation.

18. Vecteur recombinant selon l'une quelconque des revendications 1 à 17, **caractérisé en ce qu'**il est encapsidé dans une lignée psi-CRIP .

19. Vecteur recombinant selon l'une quelconque des revendications 1 à 17, **caractérisé en ce qu'**il est encapsidé dans une lignée psi-CRE.

20. Vecteur recombinant selon l'une quelconque des revendications 1 à 19, **caractérisé en ce qu'**il contient plusieurs séquences exogènes.

21. Vecteur recombinant selon l'une quelconque des revendications 1 à 20, **caractérisé en ce qu'**au moins une séquence exogène est insérée dans une séquence LTR.

22. Vecteur recombinant selon l'une quelconque des revendications 1 à 21, **caractérisé en ce que** la séquence exogène est une séquence d'intérêt thérapeutique.

23. Vecteur recombinant selon l'une quelconque des revendications 1 à 22, **caractérisé en ce que** la séquence exogène code pour un antigène ou pour un déterminant antigénique.

24. Vecteur recombinant selon l'une quelconque revendications 1 à 23 **caractérisé en ce que** la séquence exogène code pour un antigène des rétrovirus HIV, la béta-galactosidase, la gp170, la métallothionéine II, FACC, la PLP, une cytokine, le TNF, un inhibiteur tissulaire des métalloprotéinases (TIMP), l'interféron gamma, ou l'interféron beta.

25. Vecteur recombinant selon l'une quelconque des revendications 1 à 24, **caractérisé en ce que** la séquence exogène est une séquence d'ADN génomique, ou une séquence d'ADNc ou une séquence d'ARN.

26. Vecteur recombinant selon l'une quelconque des revendications 1 à 7 et 10 à 24, **caractérisé par** un titre viral supérieur ou égal à 10⁴ pfu/ml, de préférence supérieur ou égal à 10⁶ pfu/ml.

27. Cellule recombinante procaryote ou eucaryote, **caractérisée en ce qu'**elle est modifiée par un vecteur recombinant selon l'une quelconque des revendications 1 à 25, en particulier les cellules d'espèces dépourvues de rétrovirus endogènes.

28. Cellule recombinante selon la revendication 27, **caractérisée en ce qu'**il s'agit d'une cellule de mammifère, en particulier d'une cellule humaine.

29. Cellule recombinante selon la revendication 27, **caractérisée en ce qu'**il s'agit d'une cellule hématopoïétique, en particulier d'un précurseur hématopoïétique, ou d'une cellule souche totipotente lymphomyéloïde.

30. Cellule recombinante selon la revendication 27, **caractérisée en ce qu'**il s'agit d'une cellule nerveuse, notamment neuronale ou gliale, d'un fibroblaste, d'une cellule hépatique, cutanée ou musculaire, lymphocytes T ou B, ou autre médiateur de l'immunité cellulaire, de cellules tumorales, de stroma médullaire, de cellules endothéliales et mésenchymateuses.

31. Procédé pour la transfection ex *vivo* de cellules eucaryotes, **caractérisé en ce que** les cellules eucaryotes sont infectées par vecteur rétroviral recombinant selon l'une quelconque des revendications 1 à 26.

## Claims

1. Recombinant vector for the cloning and/or expression and/or transfer of an exogenous nucleotide sequence, **characterised in that** it comprises a sequence comprising the LTR sequence between nucleotides 7842 and 144 of the sequence given in figure 1, the primer fixation site (PBS) beginning at nucleotide 145, the encapsidation sequence comprised in the sequence of 250 nucleotides following the end of the LTR sequence, said sequence being capable of controlling the expression and/or transfer of the exogenous sequence, whatever its transcriptional orientation with respect to the transcriptional orientation of the virus.

2. Recombinant retroviral vector as claimed in claim 1, **characterised in that** it comprises for the expression and/or transfer of an exogenous nucleotide sequence all sequences contained in the fragment located between nucleotides 7702 to 310 of the sequence shown in figure 1, said sequence comprising sequences LTR5' and LTR3' each between nucleotides 7842 and 144, as well as the PBS site beginning at nucleotide 145, the encapsidation sequence comprised in the sequence of 250 nucleotides following the end of the LTR sequence, said sequence being capable of controlling the expression and/or transfer of the exogenous sequence, whatever its transcriptional orientation with respect to the transcriptional orientation of the virus.

3. Recombinant vector as claimed in claim 1 or claim 2, **characterised in that** it also comprises all or part of the gag sequence located between nucleotides 619 and 2245 of the sequence of figure 5, in particular the sequence between nucleotides 619 and 1527 of the sequence of figure 1.

4. Recombinant vector as claimed in claims 1 to 3, **characterised in that** it is essentially devoid of the viral sequences pol and/or env.

5. Recombinant vector as claimed in claim 1 or claim 2, **characterised in that** it comprises the fragment comprising nucleotides 7702 to 1527 of the sequence shown in figure 1.

6. Recombinant vector as claimed in any of claims 2 to 5, **characterised in that** it comprises the fragment comprising nucleotides 7702 to 310 of the sequence shown in figure 1.

7. Recombinant vector as claimed in any of claims 1 to 6, **characterised in that** it also contains at least one multiple adaptor presenting unique restriction sites with respect to the sites contained in the vector.

8. Recombinant vector as claimed in any of claims 1 to 6, **characterised in that** it is a plasmid.

9. Recombinant vector as claimed in any of claims 1 to 8, **characterised in that** it is plasmid pFOCH29 registered with the CNCM 30. June 1993 under No. I-1326.

10. Recombinant vector as claimed in any of claims 1 to 9, **characterised in that** it also comprises a marker gene or part of a marker gene, for example, the gene for resistance to neomycin.

11. Recombinant vector as claimed in any of claims 1 to 10, **characterised in that** it comprises an IRES sequence.

12. Recombinant vector as claimed in any of claims 1 to 11, **characterised in that** the U3 region of the LTR is deleted at least in part, in such a way that the promoter and/or the activator contained in U3 is (are) at least in part inactivated or modified.

13. Recombinant vector as claimed in any of claims 1 to 12, **characterised in that** the U5 region of the LTR is deleted at least in part.

14. Recombinant vector as claimed in any of claims 1 to 13, **characterised in that** the sequence contained in the fragment comprising nucleotides 7702 to 1527 and/or this fragment, and/or the sequence contained in the fragment comprising nucleotides 7702 to 310 and/or this fragment is replaced by a hybridizing sequence under conditions of high stringency with the corresponding sequence of the aforementioned fragments.

15. Recombinant vector as claimed in any of claims 1 to 14, **characterised in that** it contains the gag and pol sequences of the FB29 strain.

16. Recombinant vector as claimed in any of claims 1 to 15, **characterised in that** the exogenous nucleotide sequence is under the control of an exogenous promoter.

17. Recombinant vector as claimed in any of claims 1 to 16, **characterised in that** it is introduced in an encapsidation line.

18. Recombinant vector as claimed in any of claims 1 to 17, **characterised in that** it is encapsidated in a psi-CRIP line.

19. Recombinant vector as claimed in any of claims 1 to 17, **characterised in that** it is encapsidated in a psi-CRE line.

20. Recombinant vector as claimed in any of claims 1 to 19, **characterised in that** it contains several exogenous sequences.

21. Recombinant vector as claimed in any of claims 1 to 20, **characterised in that** at least one exogenous sequence is inserted into an LTR sequence.

22. Recombinant vector as claimed in any of claims 1 to 21, **characterised in that** the exogenous sequence is a sequence of therapeutic significance.

23. Recombinant vector as claimed in any of claims 1 to 22, **characterised in that** the exogenous sequence codes for an antigen or for an antigenic determinant.

24. Recombinant vector as claimed in any of claims 1 to 23, **characterised in that** the exogenous sequence codes for an antigen of the HIV retroviruses, beta-galactosidase, gp170, metallothioneine II, FACC, PLP, a cytokine, TNF, a tissue inhibitor of metalloproteinases (TIMP), gamma interferon, or beta interferon.

25. Recombinant vector as claimed in any of claims 1 to 24, **characterised in that** the exogenous sequence is a genomic DNA sequence, or a cDNA sequence or an RNA sequence.

26. Recombinant vector as claimed in any of claims 1 to 7 and 10 to 24, **characterised by** a viral titre greater than or equal to 10⁴ pfu/ml, preferably greater than or equal to 10⁶ pfu/ml.

27. Prokaryotic or eukaryotic recombinant cell, **characterised in that** it is modified by a recombinant vector as claimed in any of claims 1 to 25, in particular cells of species devoid of endogenous retroviruses.

28. Recombinant cell as claimed in claim 27, **characterised in that** it is a mammalian cell, in particular a human cell.

29. Recombinant cell as claimed in claim 27, **characterised in that** it is a haematopoietic cell, in particular a haematopoietic precursor, or a totipotent lymphomyeloid stem cell.

30. Recombinant cell as claimed in claim 27, **characterised in that** it is a nervous cell, in particular neuronal or glial, a fibroblast, a hepatic, cutaneous or muscular cell, T or B lymphocytes, or other mediator of cellular immunity, tumor cells, medullary stroma, endothelial and mesenchymatous cells.

31. Method for *ex vivo* transfection of eukaryotic cells, **characterised in that** the eukaryotic cells are infected by a recombinant retroviral vector as claimed in any of claims 1 to 26.

## Patentansprüche

1. Wieder kombinierender Vektor für das Klonen und/oder der Ausdruck und/oder der Transfer einer exogenen Nukleotidensequenz, charakterisiert **dadurch**, dass er eine Sequenz enthaltend die zwischen den Nukleotiden 7842 und 144 der in Abbildung 1 gegebenen Sequenz enthaltene Verkettung LTR enthält, der Befestigungspunkt des Primers (PBS) beginnend beim Nukleotiden 145, die in der Verkettung von 250 Nukleotiden folgend das Ende der Sequenz LTR enthaltene Enkapsidierungssequenz, besagte Sequenz seiend fähig den Ausdruck und/oder den Transfer der exogenen Sequenz zu kontrollieren, gleich welche ihre transkriptionelle Orientierung gegenüber der transkriptionellen Orientierung des Virus ist.

2. Wieder kombinierender retroviraler Vektor nach Anspruch 1, charakterisiert **dadurch**, dass er für den Ausdruck und/oder den Transfer einer exogenen Nukleotidensequenz jede Sequenz enthalten in dem Fragment gelegen zwischen den Nukleotiden 7702 bis 310 der in Abbildung 1 dargestellten Sequenz enthält, besagte Sequenz enthaltend die Verkettungen LTR5 und LTR3 jede enthalten zwischen den Nukleotiden 7842 und 144, sowie der Befestigungspunkt PBS beginnend beim Nukleotiden 145, die Enkapsidierungssequenz enthalten in der Verkettung von 250 Nukleotiden folgend dem Ende der Sequenz LTR, besagte Sequenz seiend fähig den Ausdruck und/oder den Transfer der exogenen Sequenz zu kontrollieren, gleich welche ihre transkriptionelle Orientierung gegenüber der transkriptionellen Orientierung des Virus ist.

3. Wieder kombinierender Vektor nach Anspruch 1 oder Anspruch 2, charakterisiert **dadurch**, dass er außerdem die gesamte oder teilweise die Sequenz gag gelegen zwischen den Nukleotiden 619 und 2245 der Sequenz von Abbildung 5 enthält, insbesondere die Sequenzen enthalten zwischen den Nukleotiden 619 und 1527 der Sequenz der Abbildung 1.

4. Wieder kombinierender Vektor nach Ansprüchen 1 bis 3, charakterisiert **dadurch**, dass ihm hauptsächlich die viralen Sequenzen pol und/oder env fehlen.

5. Wieder kombinierender Vektor nach Anspruch 1 oder Anspruch 2, charakterisiert **dadurch**, dass er das Fragment enthält, welches die Nukleotiden 7702 bis 1527 der in Abbildung 1 dargestellten Sequenz enthält.

6. Wieder kombinierender Vektor nach einem der Ansprüche 2 bis 5, charakterisiert **dadurch**, dass er das Fragment enthaltend die Nukleotide 7702 bis 310 der in Abbildung 1 dargestellten Sequenz enthält.

7. Wieder kombinierender Vektor nach einem der Ansprüche 1 bis 6, charakterisiert **dadurch**, dass er außerdem wenigstens einen multiplen Anpasser aufweisend einmalige Restriktionspunkte im Verhältnis zu den im Vektor enthaltenen Befestigungspunkten enthält.

8. Wieder kombinierender Vektor nach einem der Ansprüche 1 bis 6, charakterisiert **dadurch**, dass es sich um ein Plasmid handelt.

9. Wieder kombinierender Vektor nach einem der Ansprüche 1 bis 8, charakterisiert **dadurch**, dass es sich um den Plasmid pFOCH29 handelt, abgegeben bei der CNCM am 30 Juni 1993 unter der n°I-1326.

10. Wieder kombinierender Vektor nach einem der Ansprüche 1 bis 9, charakterisiert **dadurch**, dass er außerdem einen Gen oder einen Teil eines Markierungsgens enthält, zum Beispiel den Gen der Widerstandsfähigkeit gegenüber Neomyzin.

11. Wieder kombinierender Vektor nach einem der Ansprüche 1 bis 10, charakterisiert **dadurch**, dass er eine IRES Sequenz enthält.

12. Wieder kombinierender Vektor nach einem der Ansprüche 1 bis 11, charakterisiert **dadurch**, dass die Gegend U3 des LTR wenigstens teilweise fallen gelassen wird, auf solche Weise, dass der Initiator und/oder der Aktivator in U3 wenigstens teilweise inaktiviert oder modifiziert (wird) werden enthalten.

13. Wieder kombinierender Vektor nach einem der Ansprüche 1 bis 12, charakterisiert **dadurch**, dass wenigstens die Gegend U5 des LTR fallen gelassen wird.

14. Wieder kombinierender Vektor nach einem der Ansprüche 1 bis 13, charakterisiert **dadurch**, dass die Sequenz enthalten in dem Fragment enthaltend die Nukleotiden 7702 bis 1527 und/oder dieses Fragment, und/oder die Sequenz enthalten in dem Fragment enthaltend die Nukleotiden 7702 bis 310 und/oder dieses Fragment durch eine hybridierende Sequenz in Bedingungen starker Stringenz mit der entsprechenden Sequenz oben genannter Fragmente ersetzt wird.

15. Wieder kombinierender Vektor nach einem der Ansprüche 1 bis 14, charakterisiert **dadurch**, dass er die Sequenzen gag und pol des Ursprungs FB29 enthält.

16. Wieder kombinierender Vektor nach einem der Ansprüche 1 bis 15, charakterisiert **dadurch**, dass die Sequenz exogener Nukleotide unter der Kontrolle eines exogenen Initiators ist.

17. Wieder kombinierender Vektor nach einem der Ansprüche 1 bis 16, charakterisiert **dadurch**, dass er in eine Enkapsidationsreihe eingeführt wird.

18. Wieder kombinierender Vektor nach einem der Ansprüche 1 bis 17, charakterisiert **dadurch**, dass er in einer Reihe psi-CRIP enkapsidiert ist.

19. Wieder kombinierender Vektor nach einem der Ansprüche 1 bis 17, charakterisiert **dadurch**, dass er in einer Reihe psi-CRE enkapsidiert ist.

20. Wieder kombinierender Vektor nach einem der Ansprüche 1 bis 19, charakterisiert **dadurch**, dass er mehrere exogene Sequenzen enthält.

21. Wieder kombinierender Vektor nach einem der Ansprüche 1 bis 20, charakterisiert **dadurch**, dass wenigstens eine exogene Sequenz in eine LTR Sequenz eingeführt ist.

22. Wieder kombinierender Vektor nach einem der Ansprüche 1 bis 21, charakterisiert **dadurch**, dass die exogene Sequenz eine Sequenz von therapeutischem Interesse ist.

23. Wieder kombinierender Vektor nach einem der Ansprüche 1 bis 22, charakterisiert **dadurch**, dass die exogene Sequenz für ein Antigen oder für einen antigenischen Determinanten kodiert.

24. Wieder kombinierender Vektor nach einem der Ansprüche 1 bis 23, charakterisiert **dadurch**, dass die exogene Sequenz für ein Antigen des Retrovirus HIV, die Betagalaktosidase, die gp170, die Metallothionein II, FACC, die PLP, eine Zytokine, den TNF, einen Gewebeinhibitor der Metalloproteinasen (TIMP), das Interferon Gamma, oder das Interferon Beta kodiert.

25. Wieder kombinierender Vektor nach einem der Ansprüche 1 bis 24, charakterisiert **dadurch**, dass die exogene Sequenz eine genomische Sequenz von DNS ist, oder eine Sequenz von DNSc oder eine Sequenz von RNS ist.

26. Wieder kombinierender Vektor nach einem der Ansprüche 1 bis 7 und 10 bis 24, charakterisiert durch einen viralen Gehalt von 10⁴ pfu/ml oder mehr, vorzugsweise 10⁶ pfu/ml oder mehr.

27. Wieder kombinierende prokaryote oder eukariote Zelle, charakterisiert **dadurch**, dass sie durch einen wieder kombinierenden Vektor nach einem der Ansprüche 1 bis 25, insbesondere die Zellen von Spezies ohne einen endogenen Retrovirusmodifiziert wird.

28. Wieder kombinierende Zelle nach Anspruch 27, charakterisiert **dadurch**, dass es sich um eine Säugetierzelle handelt, insbesondere um eine menschliche Zelle.

29. Wieder kombinierende Zelle nach Anspruch 27, charakterisiert **dadurch**, dass es sich um eine hematopoïetische Zelle handelt, insbesondere ein hematopoietischer Präkursor, oder eine totipotente lymphomyeloide Stammzelle.

30. Wieder kombinierende Zelle nach Anspruch 27, charakterisiert **dadurch**, dass es sich um eine Nervenzelle handelt, insbesondere neuronal oder glial, ein Fibroblast, eine Leberzelle, Haut oder Muskelzelle, T oder B Lymphozyten, oder andere Vermittler der Zellimmunität, Tumorzellen, stoma medullaire Zellen, endotheliale und mesenchymateuse Zellen.

31. Prozedur für die ex vivo Transfektion von eukaryoten Zellen, charakterisiert **dadurch**, dass die eukaryoten Zellen durch wieder kombinierenden retroviralen Vektor nach einem der Ansprüche 1 bis 26 infiziert werden.
